(19) Europäisches Patentamt
European Patent Office
Office européen des brevets

(11) Numéro de publication: **0 034 529**
**B1**

(12) # FASCICULE DE BREVET EUROPEEN

(45) Date de publication du fascicule du brevet: **20.07.83**

(21) Numéro de dépôt: **81400204.4**

(22) Date de dépôt: **10.02.81**

(51) Int. Cl.³: **C 07 D 487/04,** A 61 K 31/505 // C07D239/95 ,(C07D487/04, 249/00, 239/00)

(54) **Nouveaux dérivés de la triazoloquinazolinone et leurs sels avec les acides, leur préparation, leur application comme médicaments, et les compositions les renfermant.**

(30) Priorité: **14.02.80 GB 8005089**

(43) Date de publication de la demande: **26.08.81 Bulletin 81/34**

(45) Mention de la délivrance du brevet: **20.07.83 Bulletin 83/29**

(84) Etats contractants désignés: **AT BE CH DE FR IT LI LU NL SE**

(56) Documents cités: **neant**

(73) Titulaire: **ROUSSEL-UCLAF, 35, boulevard des Invalides, F-75007 Paris (FR)**

(72) Inventeur: **Westwood, Robert, 8, Fernham Road, Faringdon Oxon (GB)**
Inventeur: **Tully, Wilfred Roger, 5, Street Peters Road, Cirencester Glos (GB)**
Inventeur: **Murdoch, Robert, 89, Knowlands, Highworth Wiltshire (GB)**

(74) Mandataire: **Vieillefosse, Jean-Claude et al, ROUSSEL-UCLAF Boîte postale no. 9 102, route de Noisy, F-93230 Romainville (FR)**

**EP 0 034 529 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Nouveaux dérivés de la triazoloquinazolinone et leurs sels avec les acides, leur préparation, leur application comme médicaments, et les compositions les refermant.

La présente invention concerne de nouveaux dérivés de la triazoloquinazolinone ainsi que leurs sels, leur préparation, leur application à titre de médicaments, et les compositions les refermant.

L'invention a pour objet les nouveaux dérivés de la triazoloquinazolinone, ainsi que leurs sels d'addition avec les acides minéraux ou organiques, caractérisés en ce qu'ils répondent à la formule générale I:

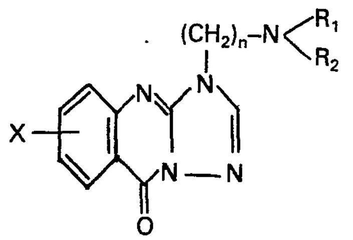

I

dans laquelle X représente un atome d'hydrogène, de fluor, de chlore ou de brome, ou un radical nitro, trifluorométhyl, méthyl ou méthoxy, n représente un nombre entier égal à 2, 3, 4 ou 5, $R_1$ et $R_2$ représentent un atome d'hydrogène, un radical alcoyl ou hydroxyalcoyl refermant de 1 à 5 atomes de carbone, ou $R_1$ et $R_2$ représentent avec l'atome d'azote un radical pyrrolidino, pipéridino, morpholino ou pipérazin-1-yl, pouvant être non substitué, ou substitué par un ou plusieurs radicaux hydroxyles, alcoyles ou hydroxy-alcoyles renfermant de 1 à 5 atomes de carbone, cycloalcoyles renfermant de 3 à 6 atomes de carbone, formyl, acétyl, carbamoyl, thiocarbamoyl, mono ou dialcoyl carbamoyl ou thiocarbamoyl les radicaux alcoyles renfermant de 1 à 6 atomes de carbone, alcoylsulfonyls les radicaux alcoyles renfermant de 1 à 6 atomes de carbone, alcoxycarbonyl renfermant de 2 à 6 atomes de carbone ou phényles, lesdits phényles pouvant eux-mêmes être non substitués ou substitués par un atome d'halogène ou un groupement trifluorométhyl.

Dans la formule générale I et dans ce qui suit, le terme radical alcoyl renfermant de 1 à 5 atomes de carbone désigne, par exemple, un radical méthyl, éthyl, propyl, isopropyl, butyl, tert.-butyl ou pentyl. Lorsque $R_1$ et $R_2$ représentent un radical hydroxyalcoyl, le radical alcoyl est, de préférence, un radical méthyl, éthyl, propyl, butyl ou pentyl, et plus particulièrement un radical éthyl.

Les radicaux pyrrolidino, pipéridino, morpholino, et pipérazin-1-yl peuvent être substitués par exemple par un ou plusieurs radicaux hydroxyles, méthyles, éthyles, hydroxyéthyles, cycloalcoyles comportant de 3 à 6 atomes de carbone, comme les radicaux cyclopropyl ou cyclohexyl, par un ou plusieurs radicaux formyl, acétyl, carbamoyl, thiocarbamoyl, mono ou dialcoyl carbamoyl ou thiocarbamoyl, les radicaux alcoyles renfermant de 1 à 6 atomes de carbone, par un ou plusieurs radicaux alcoylsulfonyls, les radicaux alcoyles renfermant de 1 à 6 atomes de carbone, par un ou plusieurs radicaux méthoxy carbonyl, éthoxycarbonyl, propoxycarbonyl, butoxycarbonyl, ou isobutoxycarbonyl, ou par un ou plusieurs radicaux phényles.

Les sels d'addition avec les acides minéraux ou organiques peuvent être, par exemple, les sels formés avec les acides chlorhydrique, bromhydrique, iodhydrique, nitrique, sulfurique, phosphorique, acétique, formique, propionique, maléique, fumarique, succinique, tartrique, citrique, oxalique, glyoxylique, aspartique, alcanesulfoniques, tels que les acides méthane ou éthanesulfoniques, arylsulfoniques, tels que les acides benzène ou paratoluènesulfoniques et arylcarboxyliques, tels que l'acide benzoïque.

Parmi les dérivés, objet de l'invention, on peut citer notamment les dérivés répondant à la formule I ci-dessus, ainsi que leurs sels d'addition avec les acides minéraux ou organiques, caractérisés en ce que, dans ladite formule I, X représente un atome d'hydrogène ou un radical méthyl ou nitro, n représente un nombre entier égal à 3, 4 ou 5, $R_1$ et $R_2$ représentent ensemble avec l'atome d'azote auquel ils sont liés un radical pyrrolidino, pipéridino, morpholino ou pipérazin-1-yl pouvant être substitué par un ou plusieurs groupements hydroxyles, cycloalcoyles renfermant de 3 à 6 atomes de carbone, alcoxycarbonyles renfermant de 2 à 6 atomes de carbone ou phényles, radicaux phényles éventuellement substitués par un atome d'halogène.

Parmi ceux-ci, on peut citer plus particulièrement les dérivés caractérisés en ce que dans ladite formule I, X représente un atome d'hydrogène ou un radical méthyle, n représente un nombre entier égal à 3, 4 ou 5, $R_1$ et $R_2$ représentent ensemble avec l'atome d'azote auquel ils sont liés, un groupe pipérazin-1-yl dont le deuxième hétéroatome est substitué par un groupement éthoxycarbonyl ou par un groupement phényle pouvant être substitué par un atome de chlore, ainsi que leurs sels d'addition avec les acides minéraux ou organiques. On peut citer tout particulièrement:

– la 1-/4-(4-m-chlorophényl pipérazin-1-yl) butyl/ triazolo /1,2,4/ /5,1-b/-quinazolin-5(1H)-one,
– la 1/4-(4-éthoxycarbonyl pipérazin-1-yl) butyl/ triazolo /1,2,4/ /5,1-b/-quinazolin-5 (1H)-one,
– la 1-/4-(4-éthoxycarbonyl pipérazin-1-yl) butyl/ 7-méthyl triazolo /1,2,4/ /5,1-b/ quinazolin-5-(1H)one,
– la 1-/3-(4-éthoxycarbonyl pipérazin-1-yl) propyl/ 7-méthyl triazolo /1,2,4/ /5,1-b/ quinazolin-5-(1H) one, ainsi que leurs sels d'addition avec les acides minéraux ou organiques.

L'invention a également pour ojet un procédé de préparation des dérivés, tels que définis par la formule I ci-dessus, ainsi que de leurs sels, caractérisé en ce que:

– soit l'on fait réagir un produit de formule générale II:

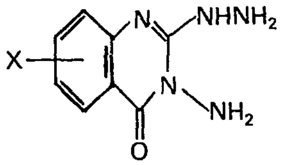
II

dans laquelle X est défini comme précédemment, avec un ω-amino alcanol de formule générale III,

$NH_2-(CH_2)_n-OH$ III

dans laquelle n a la signification déjà indiquée, pour obtenir un produit de formule générale IV:

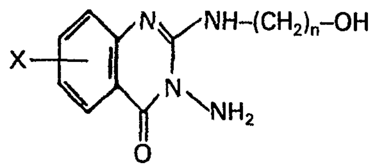
IV

dans laquelle n et X ont la signification déjà indiquée, produit de formule générale IV que l'on fiat réagir avec un agent de formylation pour obtenir un produit de formule générale V:

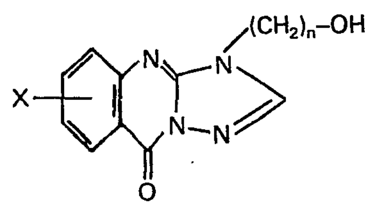
V

dans laquelle n et X ont la signification déjà indiquée, produit de formule générale V que l'on soumet à l'action d'un agent d'halogénation pour obtenir un produit de formule générale VI:

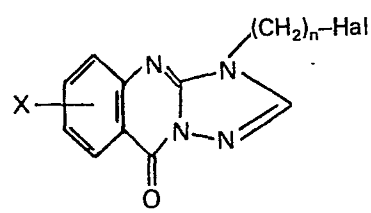
VI

dans laquelle n et X ont la signification déja indiquée et Hal représente un atome de chlore, de brome ou d'iode,

– soit l'on fait réagir un produit de formule générale VIII:

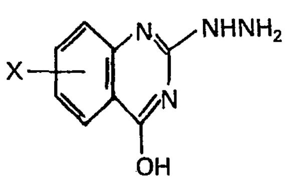
VIII

dans laquelle X a la signification déjà indiquée, avec un agent de formylation, pour obtenir un produit de formule générale IX:

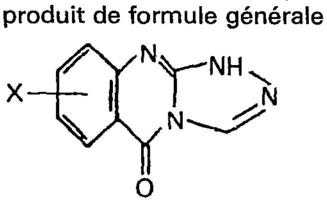
IX

dans laquelle X a la signification déjà indiquée, produit de formule générale IX que l'on traite par un agent alcalin pour obtenir un produit de formule générale X:

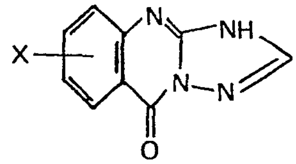
X

dans laquelle X a la signification déjà indiquée, produit de formule générale X que l'on fait réagir avec un composé de formule générale XI:

$Hal_1-(CH_2)_nHal_2$ XI

dans laquelle $Hal_1$ et $Hal_2$, identiques ou différents, représentent un atome de brome, de chlore ou d'iode et n a la signification déjà indiquée, pour obtenir un produit de formule générale VI:

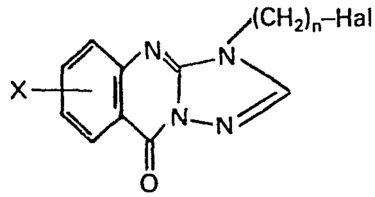
VI

dans laquelle n et X ont la signification déjà indiquée et Hal représente un atome de brome, de chlore ou d'iode, – puis enfin fait réagir le produit de formule générale VI obtenu au départ du produit de formule II au de formule VIII avec une amine de formule générale VII:

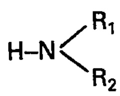
VII

dans laquelle $R_1$ et $R_2$ ont la signification déjà indiquée, pour obtenir un produit de formule générale I que l'on isole et, si désiré, salifie.

Dans des conditions préférentielles de mise en œuvre de l'invention, le procédé de préparation ci-dessus décrit est caractérisé en ce que:

– la réaction du produit de formule générale II avec l'ω-amino alcanol est effectuée à la température de 160 °C pendant 3 jours;

– l'agent de formylation que l'on fait agir avec le produit de formule IV est le diméthyl ou le diéthyl-acétal du diméthyl-formamide, la réaction étant effectuée dans un solvant hydrocarboné, de préférence le toluène, en présence d'un agent acide, de préférence l'acide paratoluène sulfonique;

– l'agent d'halogénation que l'on fait agir avec le produit de formule V est un halogénure de phosphore ou le chlorure de thionyle, la réaction étant effectuée de préférence au reflux du dichloroéthane;

– l'agent de formylation que l'on fait agir avec le produit de formule VIII est le diméthylformamide, un halogénure de benzoyle, ou, lorsque X représente un radical nitro, le triméthylorthoformiate;

– l'agent alcalin utilisé pour traiter le produit de formule générale IX est un hydroxyde alcalin, de

préférence une solution aqueuse de soude ou de potasse;

– la réaction du produit de formule X avec le produit de formule XI est effectuée en présence d'un agent basique, de préférence un hydrure alcalin ou un carbonate alcalin, et la réaction est effectuée dans l'acétone, au reflux;

– la réaction du produit de formule VI avec l'amine de formule VII est effectuée à environ 110 °C, en présence d'un solvant inerte, par exemple le diméthylformamide, et d'un agent de condensation, par exemple la pyridine ou la triéthylamine.

Les dérivés de formule I présentent un caractère basique. On peut avantageusement préparer les sels d'addition des dérivés de formule I, en faisant réagir, en proportions sensiblement stoechiométriques, un acide minéral ou organique avec ledit dérivé de formule I. Les sels peuvent être préparés sans isoler les bases correspondantes.

Les dérivés, objet de la présente invention, possèdent de très intéressantes propriétés pharmacologiques; ils sont doués notamment de remarquables propriétés antihistaminiques et bronchospasmolytiques.

Ces propriétés sont illustrées plus loin dans la partie expérimentale.

Ces propriétés justifient l'utilisation des dérivés de la triazoloquinazolinone, ainsi que de leurs sels, pharmaceutiquement acceptables, à titre de médicaments.

La présente demande a ainsi également pour objet l'application, à titre de médicaments, des dérivés de la triazoloquinazolinone, tels que définis par la formule I, ainsi que de leurs sels d'addition avec les acides pharmaceutiquement acceptables.

Parmi les médicaments, objet de l'invention, on retient, de préférence, les médicaments caractérisés en ce qu'ils sont constitués par les nouveaux dérivés de la triazoloquinazolinone répondant à la formule I, dans laquelle X représente un atome d'hydrogène ou un radical méthyl ou nitro, n représente un nombre entier égal à 3, 4 ou 5, $R_1$ et $R_2$ représentent ensemble avec l'atome d'azote auquel ils sont liés un hétérocycle saturé renfermant, si désiré, un autre hétéroatome, et pouvant être substitué par un ou plusieurs groupements hydroxyles, cycloalcoyles renfermant de 3 à 6 atomes de carbone, alcoxycarbonyles renfermant de 2 à 6 atomes de carbone ou phényles, radicaux phényles éventuellement substitués par un atome d'halogène, ainsi que par leurs sels d'addition avec les acides pharmaceutiquement acceptables.

Parmi ceux-ci, on retient notamment ceux répondant à la formule I, dans laquelle X représente un atome d'hydrogène ou un radical méthyl, n représente un nombre entier égal à 3, 4 ou 5, $R_1$ et $R_2$ représentent ensemble avec l'atome auquel ils sont liés un groupe pipérazin-1-yl dont le deuxième hétéroatome est substitué par un groupement éthoxycarbonyl ou par un groupement phényle pouvant être substitué par un atome de chlore, ainsi que leurs sels d'addition avec les acides pharmaceutiquement acceptables.

Parmi ces derniers, on retient tout particulièrement, les dérivés dont les noms suivent:

– la 1-/4-(4-m-chlorophényl pipérazin-1-yl) butyl/ triazolo /1,2,4/ /5,1-b/-quinazolin-5-(1H)-one,

– la 1/4-(4-éthoxycarbonyl pipérazin-1-yl) butyl/ triazolo /1,2,4/ /5,1-b/-quinazolin-5-(1H)-one,

– la 1-/4-(4-éthoxycarbonyl pipérazin-1-yl) butyl/ 7-méthyl triazolo /1,2,4/ /5,1-b/ quinazolin-5-(1H)one,

– la 1-/3-(4-éthoxycarbonyl pipérazin-1-yl) propyl/ 7-méthyl triazolo /1,2,4/ /5,1-b/ quinazolin-5-(1H)-one, ainsi que leurs sels d'addition avec les acides pharmaceutiquement acceptables.

Les médicaments selon l'invention trouvent leur emploi par exemple, dans le traitement de l'asthme, de la bronchite et des désordres allergiques.

La dose usuelle, variable selon le dérivé utilisé, le sujet traité et l'affection en cause peut être, par exemple de 1 mg à 1 g par jour, par voie orale chez l'homme.

L'invention a enfin pour objet les compositions pharmaceutiques qui renferment au moins un dérivé précité ou l'un de ses sels d'addition avec les acides pharmaceutiquement acceptables, à titre de principe actif.

A titre de médicaments, les dérivés répondant à la formule I et leurs sels d'addition avec les acides pharmaceutiquement acceptables peuvent être incorporés dans des compositions pharmaceutiques destinées à la voie digestive, parentérale ou locale.

Ces compositions pharmaceutiques peuvent être, par exemple, solides ou liquides et se présenter sous les formes pharmaceutiques couramment utilisées en médicine humaine, comme par exemple, les comprimés, simples ou dragéifiés, les gélules, les granulés, les aérosols, les suppositoires, les préparations injectables; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Les produits de formule II dans laquelle X a la signification déjà indiquée, lorsqu'ils ne sont pas connus, peuvent être préparés par action de l'hydrate d'hydrazine sur les 2-alcoxy 3-alcoyl quinazolin-4 (3H)-ones obtenues comme indiqué dans le brevet U.S. no 3 755 582.

Le produit de formule VIII dans laquelle X représente un radical nitro peut être obtenu en faisant réagir avec l'hydrate d'hydrazine, une solution de 2-chloro 4-hydroxyquinazoline traitée au préalable par un mélange sulfonitrique. Les autres produits de formule VIII sont préparés en traitant par l'hydrate d'hydrazine les 2-chloro-4 (3H) quinazolinones décrites par exemple par Hess dans J. MED. CHEM. Janvier 1968 vol 11 p. 135.

Les produits de formule VII dans laquelle $R_1$ et

$R_2$ représentent avec l'azote un groupement pipérazine substitué par un cycloalcoyle, lorsqu'ils ne sont pas connus, peuvent être préparés en faisant réagir la 1-benzyl pipérazine avec un halogénure du cycloalcoyle correspondant, puis en éliminant le groupement benzyle par hydrogénation catalytique.

Des exemples de telles préparations sont donnés ci-après dans la partie expérimentale.

Il va être donné maintenant, à titre non limitatif, des exemples de mise en œuvre de l'invention.

Exemple 1

Dichlorhydrate de 1-/4-/4-(m-chlorophényl) piperazin-1-yl/-butyl/-triazolo /1, 2, 4/ /5,1-b/quinazolin-5(1H)-one

Stade A: 3-amino-2-(4-hydroxybutylamino)-quinazolin-4(3H)-one.

On maintient sous agitation à 160 °C pendant 3 jours un mélange de 16 g de 3-amino 2-hydrazino quinazolin-4(3H)-one dans 48 cm3 de 4-amino butanol.

On distille ensuite sous pression réduite l'excédent de 4-amino butanol et dilue le résidu à l'eau.

On maintient ce mélange á 0 °C pendant une nuit, filtre le produit cristallisé, le lave et le sèche. On obtient 12,05 g de produit attendu.

Stade B: 1-(4-hydroxybutyl)-triazolo/1,2,4/ /5,1-b/ quinazolin-5(1H)-one

On porte au reflux un mélange de 23 g du produit obtenu au stade A, 15 g du diméthyl acétal du diméthylformamide et 2 g d'acide paratoluènesulfonique dans 230 $cm^3$ de toluène, jusqu'à cessation du dégagement de diméthyl amine.

On refroidit ensuite, filtre le produit cristallisé, le lave à l'éther et le sèche. On obtient 22,8 g de produit attendu.

Stade C: 1-(4-chlorobutyl)-triazolo/1,2,4/ /5,1-b/-quinazolin-5(1H)-one

On porte au reflux pendant six heures et demie un mélange de 23 g du produit obtenu au stade B dans 13,5 $cm^3$ de chlorure de thionyle et 500 $cm^3$ de dichloroéthane. On refroidit le mélange, ajoute un volume égal d'une solution aqueuse de carbonate de sodium et maintient sous agitation jusqu'à dissolution complète.

On sépare la phase organique, la sèche, la concentre à environ 100 $cm^3$, refroidit, ajoute 100 $cm^3$ d'éther, filtre les cristaux formés, les lave à l'éther et les sèche.

On obtient 22,5 g de produit attendu.

Stade D: Dichlorhydrate de 1-/4-/4-(m-chlorophényl) pipérazin-1-yl/butyl/-triazolo/1,2,4/5,1-b/quinazolin-5(1H)-one

On chauffe à 110 °C sous agitation pendant quinze heures un mélange de 23 g du produit obtenu au stade C, 25 g de 1-(m-chlorophényl)pipérazine et 400 $cm^3$ de diméthylformamid refroidit et dilue avec 1,5 litre de chloroforme.

On lave la solution chloroformique à l'eau, la sèche et évapore le solvant sous pression réduite. On dissout le résidu dans le méthanol et traite la solution obtenue par une solution d'acide chlorhydrique dans l'acétate d'éthyle, filtre les cristaux de chlorhydrate formés et les lave au méthanol. Le produit brut ainsi obtenu est purifié par recristallisation dans le méthanol. On obtient 31,5 g de produit attendu.

La 3-amino 2-hydrazino quinazolin-4(3H)-one peut être préparée selon le procédé suivant:

On maintient sous agitation au reflux pendant environ cinq heures de la 3-méthyl 2-thioquinazolin-4(3H)-one (demande allemande 2 539 396) ou de la 2-chloro 4-hydroxy quinazoline (J. Chem. Soc. 1947, 775) ou de la 2-hydrazino 4-hydroxyquinazoline (Bull.Soc. Chem. Belge 1959, 68, 220) dans 5 volumes d'hydrate d'hydrazine. Le mélange résultant est refroidit, dilué dans 5 volumes d'eau, puis refroidit à température ambiante. Le produit obtenu est filtré, lavé à l'eau puis au méthanol et séché. On obtient le produit attendu avec un rendement de 70 à 75%.

La 2-chloro 4-hydroxyquinazoline peut être préparée selon la méthode suivante:

a) On mélange 720 g d'urée et 1 litre de diméthylformamide, porte à 150 °C et ajoute par petites portions 978 g d'anhydride d'isatoïque. On porte la température à 180 °C jusqu'à fin de dégagement d'ammoniaque puis laisse refroidir légèrement. On ajoute 1 litre d'eau au mélange puis un litre de méthanol. On maintient pendant un heure sous agitation le mélange tiède obtenu, et la quinazoline 2,4-dione obtenue est filtrée et lavée à l'eau tiède puis au méthanol.

On peut purifier le produit par lavage au diméthylformamide filtration puis lavage au méthanol. On obtient 747 g de produit attendu.

b) On porte au reflux pendant 5 heures et demie un mélange de 300 g de quinazolin-2,4-dione, 150 $cm^3$ de N,N-diméthyl aniline et un litre de chlorure de phosphoryl, refroidit légèrement et verse sous agitation sur de la glace. Le mélange obtenu est filtré et le produit est lavé à l'eau glacée jusqu'à neutralité. Le produit ainsi obtenu est introduit dans 2,25 litres d'une solution aqueuse 2N hydroxyde de sodium et le mélange ainsi obtenu est maintenu sous agitation jusqu'à obtention de une solution claire (environ 3 heures). On filtre et le filtrat est neutralisé par de l'acide acétique. Les cristaux formés sont filtrés lavés à l'eau puis séchés. On obtient 263 g de 2-chlore 4 hydroxyquinazoline attendus.

A partir de la 2-chloro 4-hydroxyquinazoline obtenue ci-dessus, on peut obtenir un 3-amino 2-hydrazinoquinazolin-4 (3H)-one selon le procédé suivant:

On porte au reflux un mélange de 134 g de 2-chloro 4-hydroxyquinazoline dans 670 $cm^3$ d'hydrate d'hydrazine jusqu'à fin de dégagement d'ammoniaque. On laisse refroidir le mélange ainsi obtenu, le dilue par 670 $cm^3$ d'eau puis laisse refroidir à température ambiante. On filtre le produit obtenu, lave à l'eau puis au méthanol et le sèche; On obtient 101, 5 g de produit attendu.

Exemple 2

Dichlorhydrate de 1-4-/4-(m-chlorophényl) pipérazin-1-yl)-butyl/-triazolo/1,2,4/ /5,1-b/quinazoline-5 (1H)-one

Stade A: triazolo/1,2,4/3,4-b/quinazolin-5(1H)-one

On mélange 20 g de chlorure de benzoyle dans 40 $cm^3$ de diméthylformamide puis y ajoute sous agitation 10 g de 2-hydrazino 4-hydroxyquinazoline. Le mélange obtenu est porté au reflux jusqu'à obtention d'une solution claire puis versé dans l'eau. Le produit cristallisé est filtré, lavé à l'eau puis au méthanol et séché. On obtient 8 g de produit attendu.

Stade B: triazolo/1,2,4/5,1-b/quinazolin-5(1H)-one

On introduit le produit obtenu au stade A dans une solution aqueuse 0,5N d'hydroxyde de sodium puis maintient sous agitation à 90 °C jusqu'à fin de réaction. La solution obtenue est refroidie puis acidifiée par de l'acide acétique.

Les cristaux formés sont filtrés, lavés à l'eau puis au méthanol et séchés. On obtient le produit attendu avec un rendement de 100%.

Stade C: 1-(4-chlorobutyl)/1,2,4/triazolo/5,1-b/quinazolin-5(1H)-one

On fait réagir le produit obtenu au stade B avec du 4-bromochlorobutane en utilisant soit un mélange hydrure de sodium/diméthylformamide, soit un mélange utilisé au reflux d'acétone et de carbonate de potassium. Dans les 2 cas, un mélange de produit 1-substitué et 3-substitué est obtenu, ce mélange est séparé par chromatographie sur colonne d'alumine. Le produit 3-substitué est obtenu par élution avec un mélange chloroforme-éther de pétrole (1/1), puis le produit 1-substitué est obtenu avec un rendement de 30%.

Stade D: Dichlorhydrate de 1-4-/4-(m-chlorophényl) pipérazin-1-yl/-butyl-/1,2,4/triazolo/5,1-b/quinazolin-5 (1H)-one

On chauffe à 110 °C pendant quinze heures un mélange de 23 g du produit obtenu au stade C, 25 g de 1-m-chlorophényl pipérazine dans 400 $cm^3$ de diméthylformamide. On refroidit et dilue par 1,5 litre de chloroforme. La solution obtenue est lavée à l'eau, séchée puis concentrée à sec. Le résidu obtenu est dissous dans le méthanol, la solution ainsi obtenue est traitée par une solution d'acide chlorhydrique dans l'acétate d'éthyle. Les cristaux de chlorhydrate sont filtrés et lavés au méthanol. Le produit obtenu est purifié par recristallisation dans le méthanol. On obtient 31,5 g de produit attendu.

Exemple 3

Dichlorhydrate de 1-4-/4-(éthoxycarbonyl)pipérazin-1-yl) butyl-7-méthyl-/1,2,4/triazolo/5,1-b/quinazolin-5(1H)-one

Stade A: 3-amino-2-(4-hydroxybutylamino)-6-méthylquinazolin-4(3H)-one

On maintient sous agitation à 175 °C pendant 24 heures. On mélange 20 g de 3-amino 2-hydrazino-6-méthylquinazolin-4-(3H)-one dans 100 $cm^3$ de 4-aminobutanol. On distille ensuite l'excès du butanol sous pression réduite puis dilue le résidu à l'eau. Les cristaux formés sont filtrés, lavés à l'eau et séchés. On obtient 16,2 g de produit attendu.

Stade B: 1-(4-hydroxybutyl)-7-méthyl /1,2,4/triazolo/5,1-b/-quinazolin-5(1H)-one

On porte au reflux pendant environ vingt-quatre heures un mélange de 15 g du produit obtenu au stade A, 10 g de diméthyl acétal de diméthylformamide et 1,5 g d'acide paratoluénesulfonique dans 250 $cm^3$ de toluène. Le mélange est ensuite refroidi et les cristaux formés sont filtrés, lavés à l'éther puis séchés. On obtient 14 g de produit attendu.

Stade C: 1-(4-chlorobutyl)-7-méthyl/1,2,4/triazolo/5,1-b/-quinazolin-5(1H)-one.

On porte au reflux pendant 20 heures un mélange de 12 g du produit obtenu au stade B dans 12 $cm^3$ de chlorure de thionyle et 500 $cm^3$ de chloroforme. On ajoute à chaud au mélange obtenu, un volume égal d'une solution aqueuse de carbonate de sodium puis maintient sous agitation jusqu'à dissolution complète. On sépare la phase organique, la sèche et évapore le solvant.

Les cristaux formés obtenus sont lavés à l'éther puis séchés. On obtient 8,8 g de produit attendu.

Stade D

Dichlorhydrate de 1-4-/4-éthoxycarbonyl pipérazin-1-yl/butyl-7-méthyl /1,2,4/triazolo /5,1-b/quinazolin-5 (1H)-one

On chauffe à 100 °C pendant huit heures, un mélange de 8 g du produit obtenu au stade C, 8 g de pipérazine-1-carboxylate d'éthyle et 20 $cm^3$ de diméthylformamide. On dilue ensuite avec 50 $cm^3$ de chloroforme, lave la solution à l'eau, la sèche et évapore le solvant sous pression réduite. On dissout le résidu dans le méthanol puis ajoute à la solution obtenue, 8 $cm^3$ d'acide chlorhydrique concentré.

On filtre les cristaux de chlorhydrate, les lave au méthanol et les sèche. On obtient 7,6 g de produit attendu.

La 3-amino 2-hydrazino 6-méthylquinazolin-4(3H)-one utilisée au départ à l'exemple 3, peut être préparée comme suit. On porte au reflux pendant vingt-quatre heures de la 2-mercapto 3,6-diméthylquinazolin-4(3H)-one, du 2-chloro 6-méthylquinazolin-4-ol, du 2-hydrazino 6-méthylquinazolin-4-ol ou du 2-mercapto 6-méthylquinazolin-4-ol dans 5 volumes d'hydrate d'hydrazine.

Le mélange obtenu est dilué par 5 volumes d'eau puis refroidit à température ambiante. On filtre les cristaux formés, les lave à l'eau et au méthanol puis les sèche.

On obtient la 3-amino 2-hydrazino 6-méthylquinazolin-4(3H)-one avec un rendement de 95%.

Exemples 4 à 42

En utilisant une méthode analogue à celle décrite à l'exemple 1, les produits figurant dans le tableau ci-dessous ont été préparés.

La 2-hydrazino 4-hydroxy 6-nitroquinazoline qui a été utilisé au départ de l'exemple 10 peut être préparée de la manière suivante:

Stade A: 2-chloro 4-hydroxy 6-nitroquinazoline

On ajoute, goutte à goutte, à une solution refroidie et maintenue sous agitation de 90 g de 2-chloro 4-hydroxyquinazoline (J. Chem. Soc. 1947 775) dans 270 cm3 d'acide sulfurique concentré et, de telle manière, que la température ne dépasse pas 5 à 10 °C, un mélange refroidi de 70 $cm^3$ d'acide sulfurique concentré et 35 $cm^3$ d'acide nitrique concentré.

On maintient l'agitation jusqu'à ce que la température revienne à 20 °C, puis verse le mélange dans de la glace.

Les cristaux formés sont filtrés, lavés à l'eau et utilisés tels quels pour le stade suivant:

Stade B: 2-hydrazino 4 hydroxy 6-nitroquinazoline

Le produit brut obtenu au stade A est dissous dans un mélange de 90 $cm^3$ d'hydrate d'hydrazine et 900 $cm^3$ d'eau. Puis la solution obtenue est maintenue sous agitation à 90–100 °C jusqu'à cristallisation.

On refroidit le mélange obtenu, puis filtre les cristaux formés les lave à l'eau et au méthanol puis les sèche. On obtient 97,2 g de produit attendu.

| Exemple | X | n | 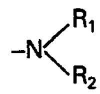 | Formule |
|---|---|---|---|---|
| 1, 2 | H | 4 | 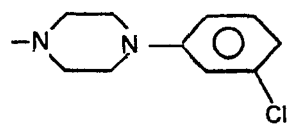 | $C_{23}H_{25}N_6OCl$, 2HCl, $H_2O$ |
| 3 | 7-Me | 4 | 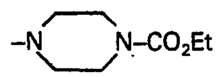 | $C_{21}H_{28}N_6O_3$ 2HCl, $^1/_2H_2$ O |
| 4 | H | 4 | 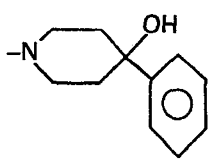 | $C_{24}H_{27}N_5O_2$, 2HCl, $H_2O$ |
| 5 | H | 4 | 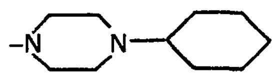 | $C_{23}H_{32}N_6O \cdot 2HCl$ |
| 6 | H | 3 | 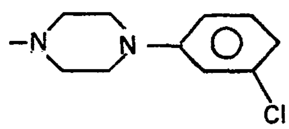 | $C_{22}H_{23}N_6OCl$, 2HCl, $2H_2O$ |
| 7 | H | 3 | 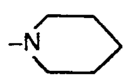 | $C_{17}H_{21}$ $N_5O$, 2HCl, $^1/_2H_2O$ |
| 8 | H | 3 | 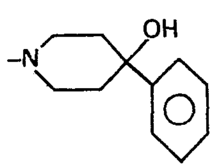 | $C_{23}H_{25}N_5O_2$, 2HCl |
| 9 | H | 3 | 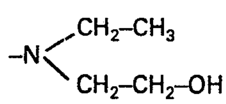 | $C_{16}H_{21}N_5O_2$, 2HCl, $^1/_2H_2O$ |
| 10 | 7–$NO_2$ | 4 | 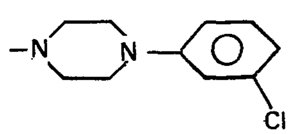 | $C_{23}H_{24}N_7O_3Cl$ |

(Fortsetzung)

| Exemple | X | n | 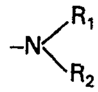 | Formule |
|---|---|---|---|---|
| 11 | H | 5 | 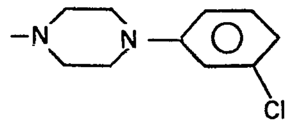 | $C_{24}H_{27}N_6OCl$, 2HCl, $1 \cdot 1/2H_2O$ |
| 12 | H | 5 | 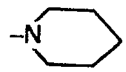 | $C_{19}H_{25}N_5O$, 2HCl, $1.1/2H_2O$ |
| 13 | $7\text{–}NO_2$ | 3 | 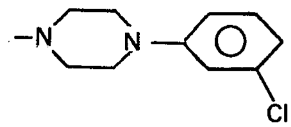 | $C_{22}H_{22}N_7O_3Cl$, HCl |
| 14 | H | 3 | 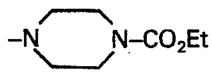 | $C_{19}H_{24}N_6O_3$ $2HCl \cdot H_2O$ |
| 15 | H | 4 | 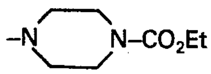 | $C_{20}H_{26}N_6O_3$ $2HCl \cdot H_2O$ |
| 16 | H | 4 | 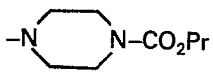 | $C_{21}H_{28}N_6O_2$ 2HCl, $1/2H_2O$ |
| 17 | H | 4 | 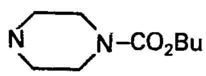 | $C_{22}H_{30}N_6O_3$ 2HCl, $1/2H_2O$ |
| 18 | H | 4 | 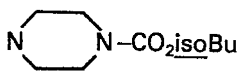 | $C_{22}H_{30}N_6O_3$ 2HCl, $1/2H_2O$ |
| 19 | H | 4 | 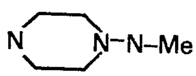 | $C_{18}H_{24}N_6O$ 2HCl, $2H_2O$ |
| 20 | H | 4 | 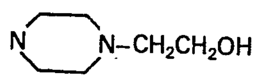 | $C_{19}H_{26}N_6O_2$ 3HCl, $1/2H_2O$ |
| 21 | H | 4 | 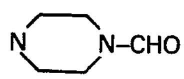 | $C_{18}H_{22}N_6O_2$ 2HCl |
| 22 | H | 4 | 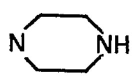 | $C_{17}H_{22}N_6O$ 2HCl |
| 23 | 7-Me | 4 | 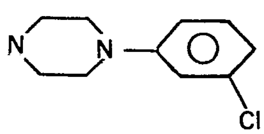 | $C_{24}H_{27}N_6OCl$ 2HCl, $H_2O$ |
| 24 | H | 4 | 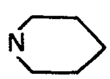 | $C_{18}H_{23}N_5O$, 2HCl, $H_2O$ |
| 25 | 7-Me | 4 | 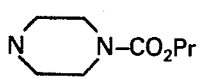 | $C_{22}H_{30}N_6O_3$ 2HCl |
| 26 | 7-Me | 4 | 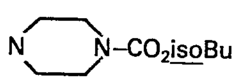 | $C_{23}H_{32}N_6O_3$ 2HCl, $1/2H_2O$ |
| 27 | 7-Me | 4 | 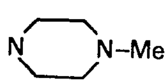 | $C_{19}H_{26}N_6O$ 3HCl, $1 \cdot 1/2H_2O$ |

(Fortsetzung)

| Exemple | X | n | 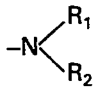 | Formule |
|---|---|---|---|---|
| 28 | 7-Me | 4 | 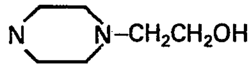 | $C_{20}H_{28}N_6O_2$ 3HCl, 1/2$H_2O$ |
| 29 | 7-Me | 4 | 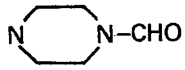 | $C_{19}H_{24}N_6O_2$ |
| 30 | 7-Me | 4 | 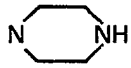 | $C_{18}H_{24}N_6O$ 3HCl, $H_2O$ |
| 31 | 7-Me | 4 | 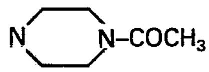 | $C_{20}H_{26}N_6O_2$ 2HCl, 1·1/2$H_2O$ |
| 32 | 8-Cl | 4 | 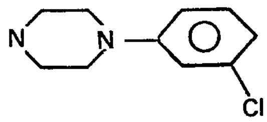 | $C_{23}H_{24}N_6OCl_2$ 2HCl |
| 33 | 8-Cl | 4 | 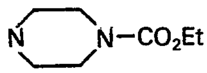 | $C_{20}H_{25}N_6O_3Cl$ HCl, ½$H_2O$ |
| 34 | 7-Me | 3 | 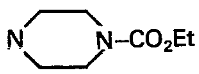 | $C_{20}H_{26}N_6O_3$ 2HCl |
| 35 | H | 4 | 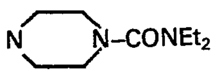 | $C_{22}H_{31}N_7O_2$ 2HCl, $H_2O$ |
| 36 | H | 4 | 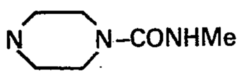 | $C_{19}H_{25}N_7O_2$ 2HCl |
| 37 | H | 4 | 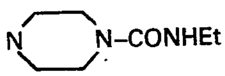 | $C_{20}H_{27}N_7O_2$ |
| 38 | H | 4 | 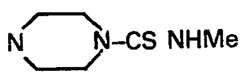 | $C_{19}H_{25}N_7OS$ 2HCl, 1/2$H_2O$ |
| 39 | H | 4 | 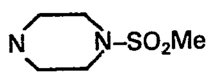 | $C_{18}H_{24}N_6O_3S$ 2HCl |
| 40 | H | 4 | 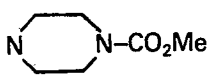 | $C_{19}H_{24}N_6O_3$ 2HCl |
| 41 | 7-Me | 4 | 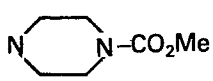 | $C_{20}H_{26}N_6O_3$ 2HCl·½$H_2O$ |
| 42 | H | 4 | 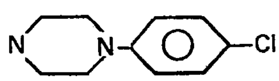 | $C_{23}H_{25}N_6O$ Cl 3HCl, ⅓$H_2O$ |

| Exemple | Calculé<br>C% | H% | Analyse Trouvé<br>Cl% | N% | F °C | Solvant de Recristallisation |
|---|---|---|---|---|---|---|
| 1, 2 | 52.33<br>52.2 | 5.57<br>5.5 | 19.67<br>19.7 | 15.92<br>15.9 | 222–5° | methanol |
| 3 | 51.01<br>50.6 | 6.32<br>6.3 | | 17.00<br>16.9 | 228–30° | |
| 4 | | 6.15<br>6.1 | 13.95<br>13.8 | 13.77<br>13.9 | 221–3° | acétate d'éthyle<br>methanol |
| 5 | | | | | 223–5° | |
| 6 | 49.68<br>49.3 | 5.45<br>5.4 | 20.00<br>19.6 | 15.79<br>15.7 | 237–8° | ethanol |
| 7 | 51.91<br>51.9 | 6.10<br>6.0 | 18.03 | 17.80<br>17.8 | 245–7° | acétate d'éthyle<br>méthanol |
| 8 | 57.99<br>57.7 | 5.71<br>5.8 | 14.88 | 14.70<br>14.7 | 210° | acétate d'éthyle<br>méthanol |
| 9 | 48.36<br>48.3 | 6.08<br>5.6 | 17.84 | 17.62<br>17.6 | 170–5° | acétate d'éthyle<br>méthanol |
| 10 | | | | | 186–9° | |
| 11 | 52.32<br>52.3 | 5.86<br>5.9 | 19.30<br>18.5 | 15.25<br>15.5 | 226–8° | acétate d'éthyle<br>méthanol |
| 12 | 51.93<br>52.0 | 6.88<br>6.7 | 16.14<br>15.4 | 15.94<br>16.3 | 216–8° | acétate d'éthyle<br>méthanol |
| 13 | 52.39<br>52.3 | 4.59<br>4.5 | 14.06<br>14.1 | 19.44<br>19.1 | 275°<br>(decomp) | |
| 14 | 48.01<br>47.9 | 5.94<br>5.6 | 14.92<br>14.5 | 17.68<br>17.6 | 222–4°<br>(decomp) | |
| 15 | 49.07<br>49.0 | 6.19<br>5.8 | 14.49<br>14.5 | 17.17<br>17.1 | 234–5°<br>(decomp) | |
| 16 | 51.02<br>51.0 | 6.32<br>6.3 | 14.34<br>14.2 | 17.00<br>17.0 | 230–2°<br>(decomp) | |
| 17 | 51.97<br>51.6 | 6.54<br>6.6 | 13.95<br>13.5 | 16.53<br>16.5 | 230–2°<br>(decomp) | |
| 18 | 51.97<br>52.2 | 6.54<br>6.5 | 13.95<br>14.0 | 16.53<br>16.7 | 230–2°<br>(decomp) | |
| 19 | 48.11<br>47.6 | 6.73<br>6.6 | 15.78<br>15.9 | 18.70<br>18.4 | 235–7° | acétate d'éthyle<br>méthanol |
| 20 | 46.68<br>46.3 | 6.19<br>5.9 | 21.76<br>21.1 | 17.19<br>17.0 | 235–7° | |
| 21 | | | | | 218–20°<br>(decomp) | |
| 22 | | | | | 220–2°<br>(decomp) | |

(Fortsetzung)

| Exemple | Calculé<br>C% | H% | Analyse<br>Trouvé<br>Cl% | N% | F °C | Solvant de Recristallisation |
|---|---|---|---|---|---|---|
| 23 | 53.48<br>53.5 | 5.25<br>5.7 | 19.73<br>19.0 | 15.60<br>15.9 | 216–9° | |
| 24 | 51.93<br>52.0 | 6.53<br>6.0 | 17.03<br>16.2 | 16.82<br>17.1 | 231–5° | |
| 26 | 52.87<br>52.9 | 6.70<br>6.5 | 13.60<br>13.3 | 16.09<br>16.1 | 244–6° | |
| 27 | 46.50<br>46.9 | 6.57<br>6.5 | | 17.12<br>17.4 | 240–2° | |
| 28 | 47.77<br>47.7 | 6.42<br>6.1 | | 16.71<br>16.7 | 244–6° | |
| 29 | 61.21<br>61.0 | 6.58<br>6.5 | | 22.55<br>22.5 | 225–7° | (Analyse sur la base libre) |
| 30 | 46.20<br>46.1 | 6.26<br>6.3 | | 17.97<br>18.0 | 250–3° | |
| 31 | 50.00<br>50.1 | 6.04<br>5.9 | | 17.50<br>17.6 | 227–230° | |
| 32 | 50.74<br>50.5 | 4.78<br>4.8 | 26.10<br>26.1 | 15.44<br>15.5 | 235–7° | |
| 33 | 50.25<br>50.7 | 5.69<br>5.7 | | 17.57<br>17.6 | 233–5° | |
| 34 | 50.95<br>50.9 | 6.00<br>6.0 | 15.04<br>15.0 | 17.83<br>17.9 | 226–8°<br>(decomp) | |
| 35 | 51.16<br>51.2 | 6.83<br>6.4 | 13.73<br>14.5 | 18.98<br>19.2 | 226–7°<br>(decomp) | |
| 36 | | | | | 203–5°<br>(decomp) | |
| 37 | 49.18<br>48.9 | 6.40<br>6.3 | | 20.07<br>20.0 | 212–5°<br>(decomp) | |
| 38 | 47.40<br>47.1 | 5.86<br>5.6 | 14.73<br>15.1 | 20.37<br>19.9 | 185–7°<br>(decomp) | |
| 39 | 42.86<br>43.0 | 5.79<br>5.7 | 14.06<br>14.1 | 16.66<br>16.9 | 220–2°<br>(decomp) | |
| 40 | | | | | 215°<br>(decomp) | acétate d'éthyle méthanol |
| 41 | 50.00<br>50.1 | 6.04<br>5.9 | | 17.50<br>17.6 | 222–5° | |
| 42 | 50.02<br>50.2 | 5.23<br>5.2 | 25.67<br>25.4 | 15.22<br>15.4 | 230–1°<br>(decomp) | |

Exemples de compositions pharmaceutiques

Exemple 43

On a préparé des comprimés ayant la formulation suivante: Dichlorhydrate de 1-[4-/4-(éthoxycarbonyl)-pipérazin-1-yl/butyl]- 7-méthyl/1,2,4/triazolo/5,1-b/quinazolin-5(1H)-one 15 mg
Excipient q.s. pour un comprimé 100 mg
(Détail de l'excipient: lactose, talc, amidon, stéarate de magnésium).

Exemple 44

On a préparé des comprimés ayant la formulation suivante: Dichlorhydrate de 1-[4-/4-(éthoxycarbonyl)- pipérazin- 1-yl/-butyl]-7-méthyl-/1,2,4/triazolo/5,1-b/quinazolin-5-(1H)-one 25 mg
Excipient q.s. pour un comprimé 100 mg
(Détail de l'excipient: lactose, talc, amidon, stéarate de magnésium).

Exemple 45

On a préparé des aérosols délivrant par dose:
Dichlorhydrate de 1-[4-/4-(éthoxycarbonyl)-pipérazin- 1-yl/-butyl]- 7-méthyl-/1,2,4/triazolo/5,1-b/quinazolin-5 (1H)-one 2 mg
Emulsifiant 0,15 mg
Propulseur 50 mg

Exemple 46

On a préparé un sirop ayant la formulation suivante: 1-[4-/4-(éthoxycarbonyl)-pipérazin-1-yl/-butyl]- 7-méthyl-/1,2,4/triazolo/5,1-b/quinazolin-5 (1H)-one 0,3 g
Excipient aromatisant q.s.p. 100 ml

Etude pharmacologique

A) Action sur le bronchospasme provoqué par l'histamine chez le cobaye.

On opère suivant la méthode de Konzett et Rossler/Arch. exp. Path. Pharmakol. 1195 71 (1940).

On utilise des cobayes mâles pesant de 300 à 400 g, à jeun depuis la veille, anestésiés par voie intrapéritonéale par de l'uréthane (0,7 ml/100 g).

On opère comme décrit par Konzett et Rossler, à l'aide d'une pompe injectant des doses d'air de 4 à 6 $cm^3$ (selon l'animal), à raison de 80 injections par minutes, afin de maintenir une surpression dans les poumons d'environ, 7,5 cm d'eau.

On mesure la pression sanguine dans l'artère carotide droite.

La substance à tester est administrée par la veine jugulaire gauche, le rinçage du dispositif étant assuré par 0,1 $cm^3$ d'une solution à 0,9% de chlorure de sodium dans l'eau distillée. La substance à tester est administrée immédiatement avant l'histamine qui est utilisée comme agoniste.

On enregistre les variations respiratoires.

Les résultats ci-dessous expriment la dose efficace de chaque produit, requise pour réduire de 50% la constriction induite par l'histamine.

| Produit de l'exemple | D.$E_{50}$ mg/Kg |
|---|---|
| 1, 2 | 0,02 |
| 3 | 0,008 |
| 4 | 0,3 |
| 5 | 0,9 |
| 6 | 0,03 |
| 7 | 0,3 |
| 8 | 2,0 |
| 10 | 0,1 |
| 11 | 0,05 |
| 12 | 1,0 |
| 15 | 0,05 |
| 24 | 0,7 |
| 34 | 0,01 |

Conclusion: Les produits des exemples 1, 2 et 3 sont particulièrement intéressants.

B) Action sur la trachée isolée de cobaye

On prélève les trachées de cobayes que l'on vient de sacrifier par un coup sur la nuque, les trachées sont découpées en spirale et suspendues dans une solution de Krebs Henseleit, à la température de 37 °C dans laquelle barbote un mélange de 95% d'oxygène et 5% de gaz carbonique.

L'histamine est utilisée comme agoniste.

Les résultats ci-dessous expriment la quantité de chaque produit testé requise pour réduire de 50% les contractions induites par l'histamine.

| Produit de l'exemple | $DE_{50}$ (ug/$cm^3$) |
|---|---|
| 1, 2 | 0,1 |
| 4 | 34 |
| 5 | 10,2 |
| 6 | 0,25 |
| 7 | 15 |
| 12 | 16 |
| 24 | 36 |

Conclusion: Le produit de l'exemple 1 ou 2 est un puissant antagoniste des contractions provoquées par l'histamine. On a de plus constaté que son action était prolongée (actif encore après 30 minutes), même après élimination du produit et lavage.

**Revendication pour les états contractants: BE, CH/LI, DE, FR, IT, LU, NL, SE**

1. Nouveaux dérivés de la triazoloquinazolinone, ainsi que leurs sels d'addition avec les acides minéraux ou organiques, caractérisés en ce que lesdits dérivés répondent à la formule générale I:

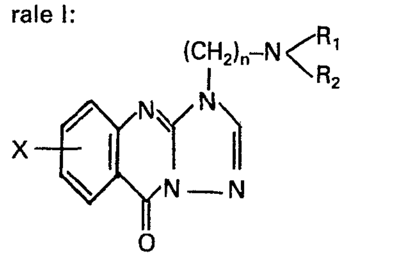

I

dans laquelle X représente un atome d'hydrogène, de fluor, de chlore, de brome, ou un radical nitro, trifluorométhyl, méthyl ou méthoxy, n représente un nombre entier égal à 2, 3, 4 ou 5, $R_1$ et $R_2$ représentent un atome d'hydrogène, un radical alcoyl ou hydroxyalcoyl renfermant de 1 à 5 atomes de carbone, ou $R_1$ et

$R_2$ représentent avec l'atome d'azote un radical pyrrolidino, pipéridino, morpholino ou pipérazin-1-yl, pouvant être non substitué, ou substitué par un ou plusieurs radicaux hydroxyles, alcoyles ou hydroxy-alcoyles renfermant de 1 à 5 atomes de carbone, cycloalcoyles renfermant de 3 à 6 atomes de carbone, formyl, acétyl, carbamoyl, thiocarbamoyl, mono ou dialcoyl carbamoyl ou thiocarbamoyl les radicaux alcoyles renfermant de 1 à 6 atomes de carbone, alcoylsulfonyls les radicaux alcoyles renfermant de 1 à 6 atomes de carbone, alcoxycarbonyl renfermant de 2 à 6 atomes de carbone ou phényles, lesdits phényles pouvant eux-mêmes être non substitués ou substitués par un atome d'halogène ou un groupement trifluorométhyl.

2. Dérivés répondant à la formule I de la revendication 1, ainsi que leurs sels d'addition avec les acides minéraux ou organiques, caractérisés en ce que dans ladite formule I, X représente un atome d'hydrogène ou un radical méthyl ou nitro, n représente un nombre entier égal à 3, 4 ou 5, $R_1$ et $R_2$ représentent ensemble avec l'atome d'azote auquel ils sont liés, un radical pyrrolidino, pipéridino, morpholino ou pipérazin-1-yl pouvant être substitué par un ou plusieurs groupements hydroxyles, cycloalcoyles renfermant de 3 à 6 atomes de carbone, alcoxycarbonyles renfermant de 2 à 6 atomes de carbone ou phényles, radicaux phényles éventuellement substitués par un atome d'halogène.

3. Dérivés répondant à la formule I de la revendication 1, ainsi que leurs sels d'addition avec les acides minéraux ou organiques, caractérisés en ce que dans ladite formule I, X représente un atome d'hydrogène ou un radical méthyl, n représente un nombre entier égal à 3, 4 ou 5, $R_1$ et $R_2$ représentent ensemble avec l'atome d'azote auquel ils sont liés, un groupe pipérazin-1-yl dont le deuxième hétéroatome est substitué par un groupement éthoxycarbonyl ou par un groupement phényle pouvant être substitué par un atome de chlore.

4. La 1/4-(4-éthoxycarbonyl pipérazin-1-yl) butyl/7-méthyl triazolo/1,2,4-/5,1-b/quinazolin-5 (1H)-one et ses sels d'addition avec les acides minéraux organiques.

5. Procédé de préparation des dérivés, tels que definis par la formule I de la revendication 1, ainsi que de leurs sels, caractérisé en ce que:

– soit l'on fait réagir un produit de formule générale II:

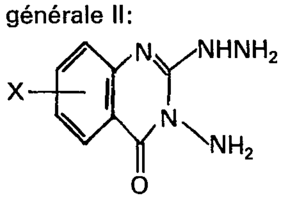

II

dans laquelle X est défini comme à la revendication 1, avec un ω-amino alcanol de formule générale III:

$NH_2-(CH_2)_n-OH$ III

dans laquelle n a la signification indiquée à la revendication 1, pour obtenir un produit de formule générale IV:

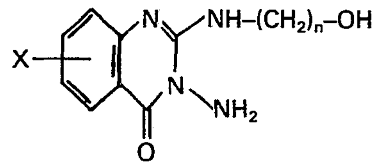

IV

dans laquelle n et X ont la signification déjà indiquée, produit de formule générale IV que l'on fait réagir avec un agent de formylation pour obtenir un produit de formule générale V:

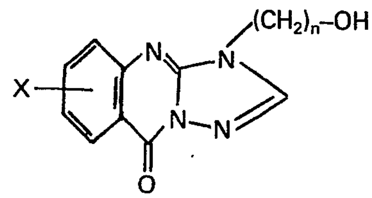

V

dans laquelle n et X ont la signification déjà indiquée, produit de formule générale V que l'on soumet à l'action d'un agent d'halogénation pour obtenir un produit de formule générale VI:

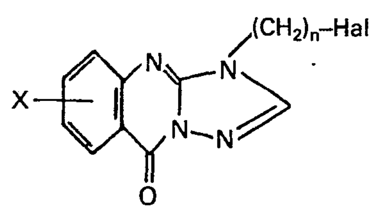

VI

dans laquelle n et X ont la signification déjà indiquée et Hal représente un atome de chlore, de brome ou d'iode,

– soit l'on fait réagir un produit de formule générale VIII:

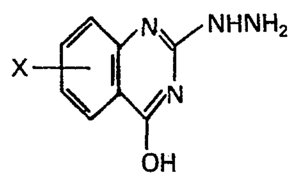

VIII

dans laquelle X a la signification déjà indiquée, avec un agent de formylation, pour obtenir un produit de formule générale IX:

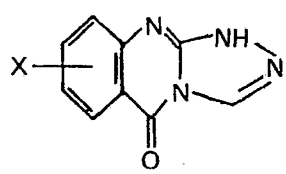

IX

dans laquelle X a la signification déjà indiquée, produit de formule générale IX que l'on traite par

un agent alcalin pour obtenir un produit de formule générale X:

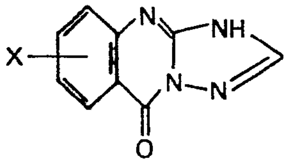

X

dans laquelle X a la signification déjà indiquée, produit de formule générale X que l'on fait réagir avec un composé de formule générale XI:

$Hal_1-(CH_2)_n Hal_2$ XI

dans laquelle $Hal_1$ et $Hal_2$, identiques ou différents, représentent un atome de brome, de chlore ou d'iode et n a la signification déjà indiquée, pour obtenir un produit de formule générale VI:

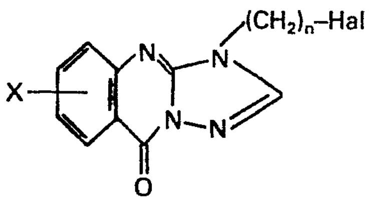

VI

dans laquelle n et X ont la signification déjà indiquée, et Hal représente un atome de brome, de chlore ou d'iode, puis enfin fait réagir le produit de formule générale VI obtenu au départ du produit de formule II ou de formule VIII avec une amine de formule générale VII:

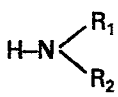

VII

dans laquelle $R_1$ et $R_2$ ont la signification indiquée à la revendication 1 pour obtenir un produit de formule générale I que l'on isole et, si désiré, salifie.

6. Procédé selon la revendication 5, caractérisé en ce que:

– l'agent de formylation que l'on fait agir avec le produit de formule IV est le diméthyl ou le diéthylacétal du diméthylformamide et la réaction est effectuée dans le toluène, en présence d'acide paratoluène sulfonique;

– l'agent d'halogénation que l'on fait agir avec le produit de formule V est un halogénure de phosphore ou le chlorure de thionyle;

– l'agent de formylation que l'on fait agir avec le produit de formule VIII est le diméthylformamide, un halogénure de benzoyle, ou, lorsque X représente un radical nitro, le triméthylorthoformiate;

– l'agent alcalin utilisé pour traiter le produit de formule générale IX est un hydroxyde alcalin;

– la réaction du produit de formule X avec le produit de formule XI est effectuée en présence d'un hydrure alcalin ou d'un carbonate alcalin, et la réaction est effectuée dans l'acétone, au reflux;

– la réaction du produit de formule VI avec l'amine de formule VII est effectuée à environ 110 °C, en présence d'un solvant inerte, et d'un agent de condensation.

7. Médicaments, caractérisés en ce qu'ils sont constitués par les nouveaux dérivés de la triazoloquinazolinone tels que définis par la formule générale I de la revendication 1, ainsi que par leurs sels d'addition avec les acides pharmaceutiquement acceptables.

8. Médicaments, caractérisés en ce qu'ils sont constitués par les nouveaux dérivés de la triazoloquinazolinone tels que définis à la revendication 2 ou 3, ainsi que par leurs sels d'addition avec les acides pharmaceutiquement acceptables.

9. Médicaments, caractérisés en ce qu'ils sont constitués par les nouveaux dérivés de la triazoloquinazolinone tels que définis à la revendication 4.

10. Compositions pharmaceutiques, caractérisées en ce qu'elles renferment à titre de principe actif, l'un au moins des médicaments tels que définis à l'une des revendications 7, 8 ou 9.

**Revendications pour l'état contractant: AT**

1. Procédé pour préparer les nouveaux dérivés de la triazoloquinazolinone, ainsi que leurs sels d'addition avec les acides minéraux ou organiques, lesdits dérivés répondant à la formule générale I:

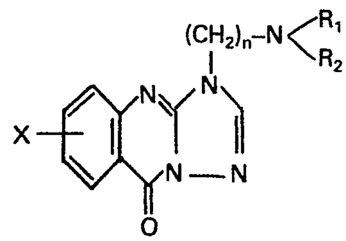

I

dans laquelle X représente un atome d'hydrogène, de fluor, de chlore, de brome, ou un radical nitro, trifluorométhyl, méthyl ou méthoxy, n représente un nombre entier égal à 2, 3, 4 ou 5, $R_1$ et $R_2$ représentent un atome d'hydrogène, un radical alcoyl ou hydroxyalcoyl renfermant de 1 à 5 atomes de carbone, ou $R_1$ et

$R_2$ représentent avec l'atome d'azote un radical pyrrolidino, pipéridino, morpholino ou pipérazin-1-yl, pouvant être non substitué, ou substitué par un ou plusieurs radicaux hydroxyles, alcoyles ou hydroxy-alcoyles renfermant de 1 à 5 atomes de carbone, cycloalcoyles renfermant de 3 à 6 atomes de carbone, formyl, acétyl, carbamoyl, thiocarbamoyl, mono ou dialcoyl carbamoyl ou thiocarbamoyl les radicaux alcoyles renfermant de 1 à 6 atomes de carbone, alcoylsulfonyls les radicaux alcoyles renfermant de 1 à 6 atomes de carbone, alcoxycarbonyl renfermant de 2 à 6 atomes de carbone ou phényles, lesdits phényles pouvant eux-mêmes être non substitués ou substitués par un atome d'halogène ou un groupement trifluorométhyl, caractérisé en ce que:

– soit l'on fait réagir un produit de formule générale II:

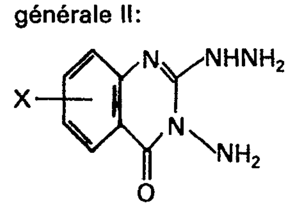

II

dans laquelle X est défini comme précédemment, avec un ω-amino alcanol de formule générale III:

$NH_2-(CH_2)_n-OH$ III

dans laquelle n a la signification indiquée précédemment, pour obtenir un produit de formule générale IV:

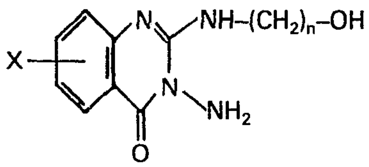
IV

dans laquelle n et X ont la signification déjà indiquée, produit de formule générale IV que l'on fait réagir avec un agent de formylation pour obtenir un produit de formule générale V:

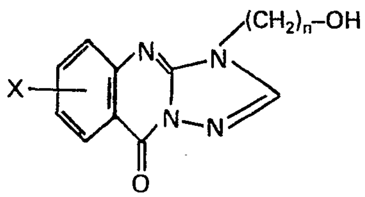
V

dans laquelle n et X ont la signification déjà indiquée, produit de formule générale V que l'on soumet à l'action d'un agent d'halogénation pour obtenir un produit de formule générale VI:

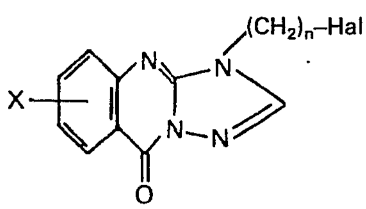
VI

dans laquelle n et X ont la signification déjà indiquée et Hal représente un atome de chlore, de brome ou d'iode, – soit l'on fait réagir un produit de formule générale VIII:

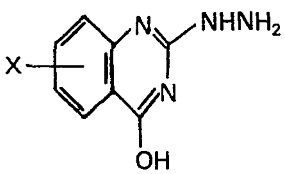
VIII

dans laquelle X a la signification déjà indiquée, avec un agent de formylation, pour obtenir un produit de formule générale IX:

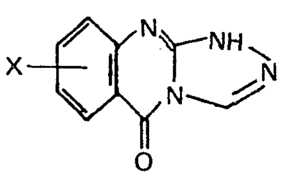
IX

dans laquelle X a la signification déjà indiquée, produit de formule générale IX que l'on traite par un agent alcalin pour obtenir un produit de formule générale X:

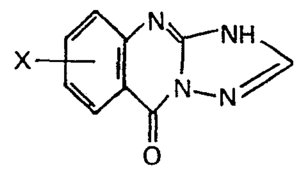
X

dans laquelle X a la signification déjà indiquée, produit de formule générale X que l'on fait réagir avec un composé de formule générale XI:

$Hal_1-(CH_2)_n\,Hal_2$ XI

dans laquelle $Hal_1$ et $Hal_2$, identiques ou différents, représentent un atome de brome, de chlore ou d'iode et n a la signification déjà indiquée, pour obtenir un produit de formule générale VI:

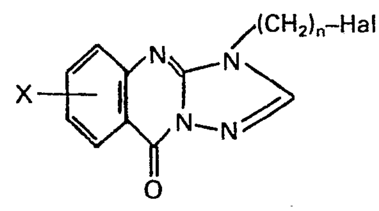
VI

dans laquelle n et X ont la signification déjà indiquée, et Hal représente un atome de brome, de chlore ou d'iode, puis enfin fait réagir le produit de formule générale VI obtenu au départ du produit de formule II ou de formule VIII avec une amine de formule générale VII:

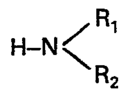
VII

dans laquelle $R_1$ et $R_2$ ont la signification déjà indiquée, pour obtenir un produit de formule générale I que l'on isole et, si désiré, salifie.

2. Procédé selon la revendication 1, caractérisé en ce que:

– l'agent de formylation que l'on fait agir avec le produit de formule IV est le diméthyl ou le diéthylacétal du diméthylformamide et la réaction est effectuée dans le toluène, en présence d'acide paratoluène sulfonique;

– l'agent d'halogénation que l'on fait agir avec le produit de formule V est un halogénure de phosphore ou le chlorure de thionyle;

– l'agent de formylation que l'on fait agir avec le produit de formule VIII est le diméthylformamide, un halogénure de benzoyle, ou, lorsque X représente un radical nitro, le triméthylorthoformiate;

– l'agent alcalin utilisé pour traiter le produit de formule générale IX est un hydroxyde alcalin;

– la réaction du produit de formule X avec le produit de formule XI est effectuée en présence d'un hydrure alcalin ou d'un carbonate alcalin, et la réaction est effectuée dans l'acétone, au reflux;

– la réaction du produit de formule VI avec l'amine de formule VII est effectuée à environ 110 °C, en présence d'un solvant inerte, et d'un agent de condensation.

3. Procédé selon la revendication 1 ou 2, caractérisé en ce que l'on utilise au départ un produit deformule II ou VIII, dans laquelle X représente un atome d'hydrogène ou un radical méthyl ou nitro,

un produit de formule III ou XI, dans laquelle n représente un nombre entier égal à 3, 4 ou 5, et un produit de formule VII dans laquelle $R_1$ et $R_2$ représentent ensemble avec l'atome d'azote auquel ils sont liés, un radical pyrrolidino, pipéridino, morpholino ou pipérazin-1-yl pouvant être substitué par un ou plusieurs groupements hydroxyles, cycloalcoyles renfermant de 3 à 6 atomes de carbone, alcoxycarbonyles renfermant de 2 à 6 atomes de carbone ou phényles, radicaux phényles, éventuellement substitués par un atome d'halogène.

4. Procédé selon la revendication 1 ou 2, caractérisé en ce que l'on utilise au départ un produit de formule II ou VIII, dans laquelle X représente un atome d'hydrogène ou un radical méthyl, un produit de formule III ou XI, dans laquelle n représente un nombre entier égal à 3, 4 ou 5 et un produit de formule VII dans laquelle $R_1$ et $R_2$ représentent ensemble avec l'atome d'azote auquel ils sont liés, un groupe pipérazin-1-yl dont le deuxième hétéroatome est substitué par un groupement éthoxycarbonyl ou par un groupement phényle pouvant être substitué par un atome de chlore.

5. Procédé selon la revendication 1 ou 2, caractérisé en ce que l'on utilise au départ des produits de formule II, III, VII, choisies de manière telle que l'on prépare le dérivé répondant à la formule I de la revendication 1, dont le nom suit:

– la 1/4-(4-éthoxycarbonyl pipérazin-1-yl) butyl/ 7-méthyl triazolo/1,2,4/ /5,1-b/-quinazolin-5(1H)-one, ainsi que ses sels d'addition avec les acides minéraux ou organiques.

**Patentansprüche für die Vertragsstaaten: BE, CH/ LI, DE, FR, IT, LU, NL, SE**

1. Neue Triazolochinazolinon-Derivate sowie deren Additionssalze mit Mineral- oder organischen Säuren, dadurch gekennzeichnet, dass diese Derivate der allgemeinen Formel I

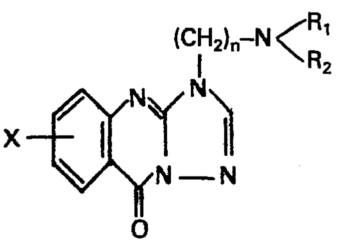

I

entsprechen, worin X ein Wasserstoff-, Fluor-, Chlor- oder Bromatom oder einen Nitro-, Trifluormethyl-, Methyl- oder Methoxyrest bedeutet, n eine ganze Zahl entsprechend 2, 3, 4 oder 5 darstellt, $R_1$ und $R_2$ ein Wasserstoffatom, einen Alkyl- oder Hydroxyalkylrest mit 1 bis 5 Kohlenstoffatomen bedeuten oder $R_1$ und $R_2$ mit dem Stickstoffatom einen Pyrrolidino-, Piperidino-, Morpholino- oder Piperazin-1-yl-rest darstellen, der unsubstituiert sein kann oder substituiert sein kann durch ein oder mehrere Hydroxylreste, Alkyl- oder Hydroxyalkylreste mit 1 bis 5 Kohlenstoffatomen, Cycloalkylreste mit 3 bis 6 Kohlenstoffatomen, Formyl-, Acetyl-, Carbamoyl-, Thiocarbamoyl-, Mono- oder Dialkylcarbamoyl- oder -thiocarbamoylreste mit 1 bis 6 Kohlenstoffatomen in den Alkylresten, Alkylsulfonylreste mit 1 bis 6 Kohlenstoffatomen in den Alkylresten, Alkoxycarbonylreste mit 2 bis 6 Kohlenstoffatomen oder Phenylreste, wobei die Phenylreste ihrerseits unsubstituiert sein können oder substituiert sein können durch ein Halogenatom oder eine Trifluormethylgruppe.

2. Derivate der Formel I gemäss Anspruch 1 sowie deren Additionssalze mit Mineral- oder organischen Säuren, dadurch gekennzeichnet, dass in der Formel I X ein Wasserstoffatom oder einen Methyl- oder Nitrorest bedeutet, n eine ganze Zahl entsprechend 3, 4 oder 5 bedeutet, $R_1$ und $R_2$ gemeinsam mit dem Stickstoffatom, an das sie gebunden sind, einen Pyrrolidino-, Piperidino-, Morpholino- oder Piperazin-1-yl-rest bilden, der substituiert sein kann durch ein oder mehrere Hydroxylgruppen, Cycloalkylgruppen mit 3 bis 6 Kohlenstoffatomen, Alkoxycarbonylgruppen mit 2 bis 6 Kohlenstoffatomen oder Phenylreste, wobei die Phenylreste gegebenenfalls durch ein Halogenatom substituiert sind.

3. Derivate der Formel I gemäss Anspruch 1, sowie deren Additionssalze mit Mineral- oder organischen Säuren, dadurch gekennzeichnet, dass in der Formel I X ein Wasserstoffatom oder einen Methylrest bedeutet, n eine ganze Zahl entsprechend 3, 4 oder 5 bedeutet, $R_1$ und $R_2$ gemeinsam mit dem Stickstoffatom, an das sie gebunden sind, eine Piperazin-1-yl-gruppe darstellen, dessen zweites Hereroatom durch eine Äthoxycarbonylgruppe oder durch eine Phenylgruppe, die durch ein Chloratom substituiert sein kann, substituiert ist.

4. 1-[4-(-Äthoxycarbonylpiperazin-1-yl)-butyl]-7-methyltriazolo-[1.2.4]-[5,1-b]-chinazolin-5(1H)-on und dessen Additionssalze mit Mineral- oder organischen Säuren.

5. Verfahren zur Herstellung der Derivate der Formel I gemäss Anspruch 1 sowie von deren Salzen, dadurch gekennzeichnet, dass man:

entweder ein Produkt der allgemeinen Formel II

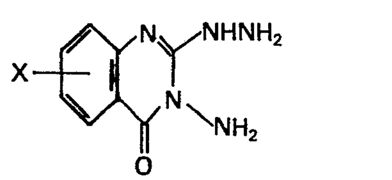

II

worin X wie in Anspruch 1 definiert ist, mit einem ω-Aminoalkanol der allgemeinen Formel III

$NH_2-(CH_2)_n-OH$ III

worin n die in Anspruch 1 angegebene Bedeutung besitzt, umsetzt, um ein Produkt der allgemeinen Formel IV

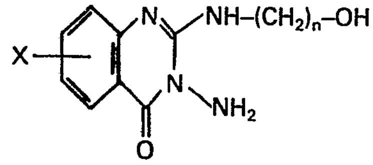

IV

worin n und X die angegebene Bedeutung besitzen, zu erhalten, welches Produkt der allgemeinen Formel IV man mit einem Formylierungsmittel umsetzt, um ein Produkt der allgemeinen Formel V

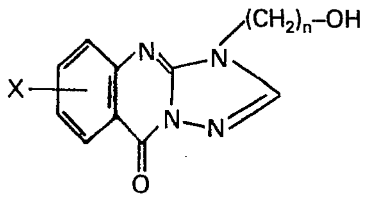

V

zu erhalten, worin n und X die angegebene Bedeutung besitzen, welches Produkt der allgemeinen Formel V man der Einwirkung eines Halogenierungsmittels unterzieht, um ein Produkt der allgemeinen Formel VI

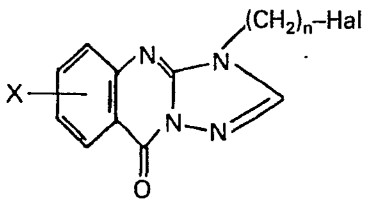

VI

zu erhalten, worin n und X die angegebene Bedeutung besitzen und Hal ein Chlor-, Brom- oder Jodatom bedeutet, oder ein Produkt der allgemeinen Formel VIII

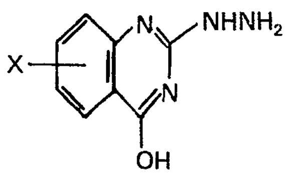

VIII

worin X die angegebene Bedeutung besitzt, mit einem Formylierungsmittel umsetzt, um ein Produkt der allgemeinen Formel IX

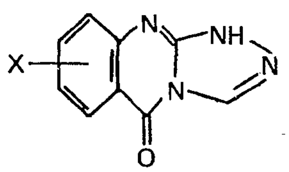

IX

worin X die angegebene Bedeutung besitzt, zu erhalten, welches Produkt der allgemeinen Formel IX man mit einem alkalischen Mittel behandelt, um ein Produkt der allgemeinen Formel X

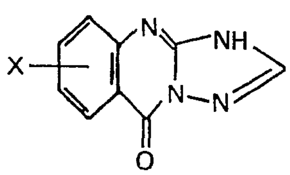

X

zu erhalten, worin X die angegebene Bedeutung besitzt, welches Produkt der allgemeinen Formel X man mit einer Verbindung der allgemeinen Formel XI

$Hal_1-(CH_2)_n-Hal_2$ XI

worin $Hal_1$ und $Hal_2$, die gleich oder verschieden sein können, ein Brom-, Chlor- oder Jodatom bedeuten und n die angegebene Bedeutung besitzt, umsetzt, um ein Produkt der allgemeinen Formel VI

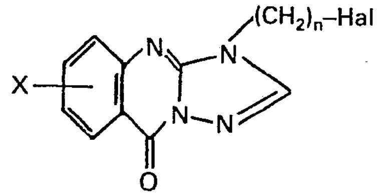

VI

zu erhalten, worin n und X die angegebene Bedeutung besitzen und Hal ein Brom-, Chlor- oder Jodatom bedeuten, und danach schliesslich das ausgehend von dem Produkt der Formel II oder der Formel VIII erhaltene Produkt der allgemeinen Formel VI mit einem Amin der allgemeinen Formel VII

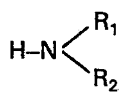

VII

worin $R_1$ und $R_2$ die in Anspruch 1 angegebene Bedeutung besitzen, umsetzt, um ein Produkt der allgemeinen Formel I zu erhalten, das man isoliert und gewünschtenfalls in ein Salz überführt.

6. Verfahren gemäss Anspruch 5, dadurch gekennzeichnet, dass: das Formylierungsmittel, das man mit dem Produkt der Formel IV umsetzt, das Dimethyl- oder Diäthylacetal des Dimethylformamids ist und die Umsetzung in Toluol in Gegenwart von p-Toluolsulfonsäure durchgeführt wird, das Halogenierungsmittel, das man mit dem Produkt der Formel V umsetzt, ein Phosphorhalogenid oder Thionylchlorid ist, das Formylierungsmittel, das man mit dem Produkt der Formel VIII umsetzt, Dimethylformamid, ein Benzoylhalogenid oder, wenn X einen Nitrorest bedeutet, Trimethylorthoformiat ist, das für die Behandlung des Produkts der allgemeinen Formel XI verwendete alkalische Mittel ein Alkalihydroxid ist, die Umsetzung des Produkts der Formel X mit dem Produkt der Formel XI in Gegenwart eines Alkylhydrids oder eines Alkalicarbonats durchgeführt wird und die Umsetzung in Aceton unter Rückfluss durchgeführt wird, die Umsetzung des Produkts der Formel VI mit dem Amin der Formel VII bei etwa 110 °C in Gegenwart eines inerten Lösungsmittels und eines Kondensationsmittels durchgeführt wird.

7. Arzneimittel, dadurch gekennzeichnet, dass sie aus den neuen Triazolochinazolinon-Derivaten der allgemeinen Formel I oder aus den Additionssalzen mit pharmazeutisch verträglichen Säuren bestehen.

8. Arzneimittel, dadurch gekennzeichnet, dass sie aus den neuen Triazolochinazolinon-Derivaten gemäss Anspruch 2 oder 3 oder aus deren Additionssalzen mit pharmazeutisch verträglichen Säuren bestehen.

9. Arzneimittel, dadurch gekennzeichnet, dass sie aus den neuen Triazolochinazolinon-Derivaten gemäss Anspruch 4 bestehen.

10. Pharmazeutische Zusammensetzungen, dadurch gekennzeichnet, dass sie als Wirkstoff zumindest eines der Arzneimittel gemäss einem der Ansprüche 7, 8 oder 9 enthalten.

**Patentansprüche für den Vertragsstaat: AT**

1. Verfahren zur Herstellung von neuen Triazolochinazolinon-Derivaten sowie von deren Additionssalzen mit Mineral- oder organischen Säuren, wobei diese Derivate der allgemeinen Formel I

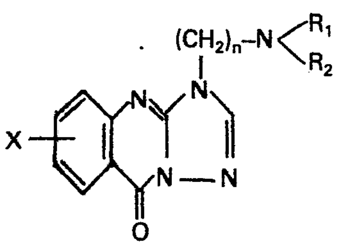

I

entsprechen, worin X ein Wasserstoff-, Fluor-, Chlor- oder Bromatom oder einen Nitro-, Trifluormethyl-, Methyl- oder Methoxyrest bedeutet, n eine ganze Zahl entsprechend 2, 3, 4 oder 5 darstellt, $R_1$ und $R_2$ ein Wasserstoffatom, einen Alkyl- oder Hydroxyalkylrest mit 1 bis 5 Kohlenstoffatomen darstellen oder $R_1$ und $R_2$ mit dem Stickstoffatom einen Pyrrolidino-, Piperidino-, Morpholino- oder Piperazin-1-yl-rest darstellen, der unsubstituiert sein kann oder substituiert sein kann durch ein oder mehrere Hydroxylreste, Alkyl- oder Hydroxyalkylreste mit 1 bis 5 Kohlenstoffatomen, Cycloalkylreste mit 3 bis 6 Kohlenstoffatomen, Formyl-, Acetyl-, Carbamoyl-, Thiocarbamoyl-, Mono- oder Dialkylcarbamoyl- oder -thiocarbamoylreste mit 1 bis 6 Kohlenstoffatomen in den Alkylresten, Alkylsulfonylreste mit 1 bis 6 Kohlenstoffatomen in den Alkylresten, Alkoxycarbonylreste mit 2 bis 6 Kohlenstoffatomen oder Phenylreste, wobei die Phenylreste ihrerseits unsubstituiert sein können oder durch ein Halogenatom oder eine Trifluormethylgruppe substituiert sein können, dadurch gekennzeichnet, dass man entweder ein Produkt der allgemeinen Formel II

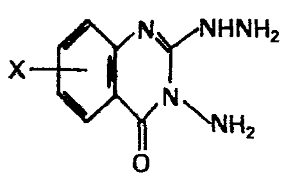

II

worin X wie vorstehend definiert ist, mit einem ω-Aminoalkanol der allgemeinen Formel III

$NH_2-(CH_2)_n-OH$ III

worin n die vorstehend angegebene Bedeutung besitzt, umsetzt, um ein Produkt der allgemeinen Formel IV

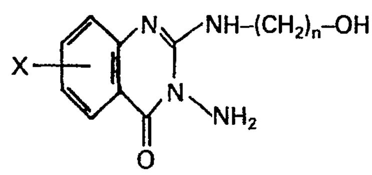

IV

zu erhalten, worin n und X die angegebene Bedeutung besitzen, welches Produkt der allgemeinen Formel IV man mit einem Formylierungsmittel umsetzt, um ein Produkt der allgemeinen Formel V

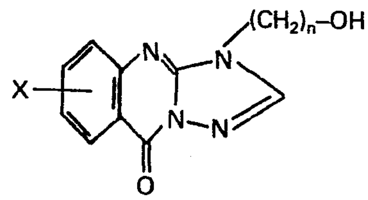

V

zu erhalten, worin n und X die angegebene Bedeutung besitzen, welches Produkt der allgemeinen Formel V man der Einwirkung eines Halogenierungsmittels unterzieht, um ein Produkt der allgemeinen Formel VI

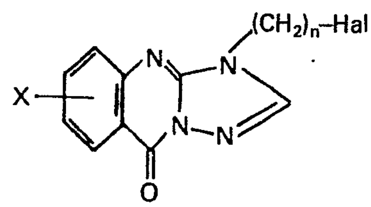

VI

zu erhalten, worin n und X die angegebene Bedeutung besitzen und Hal ein Chlor-, Brom- oder Jodatom bedeutet, oder ein Produkt der allgemeinen Formel VIII

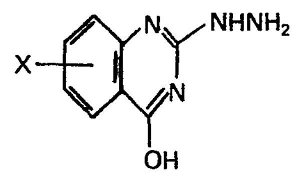

VIII

worin X die angegebene Bedeutung besitzt, mit einem Formylierungsmittel umsetzt, um ein Produkt der allgemeinen Formel IX

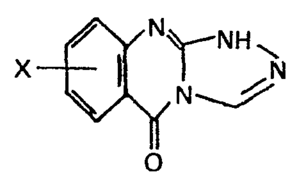

IX

worin X die angegebene Bedeutung besitzt, zu erhalten, welches Produkt der allgemeinen Formel IX man mit einem alkalischen Mittel behandelt, um ein Produkt der allgemeinen Formel X

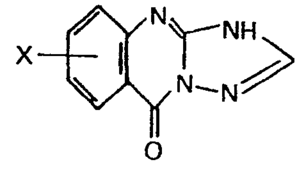

X

zu erhalten, worin X die angegebene Bedeutung besitzt, welches Produkt der allgemeinen Formel X man mit einer Verbindung der allgemeinen Formel XI

$Hal_1-(CH_2)_n-Hal_2$ XI

umsetzt, worin $Hal_1$ und $Hal_2$, die gleich oder verschieden sein können, ein Brom-, Chlor- oder Jodatom darstellen und n die angegebene Bedeutung besitzt, um ein Produkt der allgemeinen Formel VI

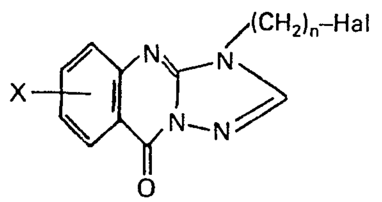

VI

worin n und X die angegebene Bedeutung besitzen und Hal ein Brom-, Chlor- oder Jodatom bedeutet, zu erhalten, wonach man schliesslich das ausgehend von dem Produkt der Formel II oder der Formel VIII erhaltene Produkt der allgemeinen Formel VI mit einem Amin der allgemeinen Formel VII

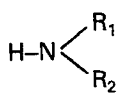

VII

worin $R_1$ und $R_2$ die angegebene Bedeutung besitzen, umsetzt, um ein Produkt der allgemeinen Formel I zu erhalten, welches man isoliert und gewünschtenfalls in ein Salz überführt.

2. Verfahren gemäss Anspruch 1, dadurch gekennzeichnet, dass: das Formylierungsmittel, das man mit dem Produkt der Formel IV umsetzt, das Dimethyl- oder Diäthylacetal des Dimethylformamids ist und die Umsetzung in Toluol in Gegenwart von p-Toluolsulfonsäure durchgeführt wird, das Halogenierungsmittel, das man mit dem Produkt der Formel V umsetzt, ein Phosphorhalogenid oder Thionylchlorid ist, das Formylierungsmittel, das man mit dem Produkt der Formel VIII umsetzt, Dimethylformamid, ein Benzoylhalogenid oder, wenn X einen Nitrorest bedeutet, Trimethylorthoformiat ist, das zur Behandlung des Produkts der allgemeinen Formel IX verwendete alkalische Mittel ein Alkalihydroxid ist, die Umsetzung des Produkts der Formel X mit dem Produkt der Formel XI in Gegenwart eines Alkalihydrids oder eines Alkalicarbonats durchgeführt wird und die Umsetzung in Aceton unter Rückfluss durchgeführt wird, die Umsetzung des Produkts der Formel VI mit dem Amin der Formel VII bei etwa 110 °C in Gegenwart eines inerten Lösungsmittels und eines Kondensationsmittels durchgeführt wird.

3. Verfahren gemäss Anspruch 1 oder 2, dadurch gekennzeichnet, dass man ausgeht von einem Produkt der Formel II oder VIII, worin X ein Wasserstoffatom oder einen Methyl- oder Nitrorest bedeutet, einem Produkt der Formel III oder XI, worin n eine ganze Zahl entsprechend 3, 4 oder 5 bedeutet und einem Produkt der Formel VII, worin $R_1$ und $R_2$ gemeinsam mit dem Stickstoffatom, an das sie gebunden sind, einen Pyrrolidino-, Piperidino-, Morpholino- oder Piperazin-1-ylrest darstellen, der substituiert sein kann durch ein oder mehrere Hydroxylgruppen, Cycloalkylgruppen mit 3 bis 6 Kohlenstoffatomen, Alkoxycarbonylgruppen mit 2 bis 6 Kohlenstoffatomen oder Phenylgruppen, wobei die Phenylgruppen gegebenenfalls durch ein Halogenatom substituiert sind.

4. Verfahren gemäss Anspruch 1 oder 2, dadurch gekennzeichnet, dass man ausgeht von einem Produkt der Formel II oder VIII, worin X ein Wasserstoffatom oder einen Methylrest bedeutet, einem Produkt der Formel III oder XI, worin n eine ganze Zahl entsprechend 3, 4 oder 5 bedeutet und einem Produkt der Formel VII, worin $R_1$ und $R_2$ gemeinsam mit dem Stickstoffatom, an das sie gebunden sind, eine Piperazin-1-yl-gruppe darstellen, dessen zweites Heteroatom durch eine Äthoxycarbonylgruppe oder durch eine Phenylgruppe, die durch ein Chloratom substituiert sein kann, substituiert ist.

5. Verfahren gemäss Anspruch 1 oder 2, dadurch gekennzeichnet, dass man ausgeht von Produkten der Formel II, III und VII, die derart ausgewählt sind, dass man das der Formel I gemäss Anspruch 1 entsprechende Derivat mit der folgenden Bezeichnung herstellt:

1-[4-(4-Äthoxycarbonylpiperazin-1-yl) -butyl]-7-methyltriazolo- [1.2.4]-[5,1-b]-chinazolin-5 (1H)-on sowie dessen Additionssalze mit Mineral- oder organischen Säuren.

**Claims for the contracting states: BE, CH/LI, DE, FR, IT, LU, NL, SE.**

1. New derivatives of triazoloquinazolinone, as well as their malts of addition with mineral or organic acid, characterized in that the said derivatives respond to the general formula I:

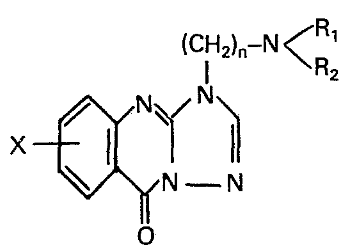

I

in which X represents a hydrogen, fluorine, chlorine, bromine atom, or a nitro, trifluoromethyl, methyl or methoxy radical, n represents a whole number 2, 3, 4 or 5, $R_1$ und $R_2$ represent a hydrogen atom, an alkyl or hydroxyalkyl radical containing from 1 to 5 carbon atoms, or $R_1$ and $R_2$ represent, with the nitrogen atom, a pyrrolidino, piperidino, morpholino or piperazin-1-yl radical, which may be unsubstituted, or substituted by one or more hydroxyl, alkyl or hydroxy-alkyl radicals containing from 1 to 5 carbon atoms, cycloalkyl radicals containing from 3 to 6 carbon atoms, formyl, acetyl, carbamoyl, thiocarbamoyl, mono

or dialkyl carbamoyl or thiocarbamoyl radicals, the alkyl radicals containing from 1 to 6 carbon atoms, alkylsulphonyl radicals, the alkyl radicals containing from 1 to 6 carbon atoms, alkoxycarbonyl radicals containing from 1 to 6 carbon atoms, alkoxycarbonyl radicals containing from 2 to 6 carbon atoms, or phenyls, the said phenyls being able to be unsubstituted or substituted by a halogen atom or a trifluoromethyl group.

2. Derivatives responding to formula I of claim 1, as well as their salts of addition with mineral or organic acids, characterized in that in the said formula I, X represents a hydrogen atom or a methyl or nitro radical, n represents a whole number, 3, 4 or 5, $R_1$ and $R_2$, together with the nitrogen atom to which they are attached, represent a pyrrolidino, piperidino, morpholino or piperazin-1-yl radical, which may be substituted by one or more hydroxyl, cycloalkyl containing from 3 to 6 carbon atoms, alkoxycarbonyl containing from 2 to 6 carbon atoms or phenyl groups, the phenyl radicals possibly being substituted by a halogen atom.

3. Derivatives responding to formula I of claim 1, as well as their salts of addition with mineral or organic acids, characterized in that in the said formula I, X represents a hydrogen atom or a methyl radical, n represents a whole number, 3, 4 or 5, $R_1$ and $R_2$ represent, together with the nitrogen atom to which they are attached, a piperazin-1-yl group, the second heteroatom of which is substituted by an ethoxycarbonyl group or by a phenyl group which may be substituted by a chlorine atom.

4. 1[4-(4-ethoxycarbonyl piperazin-1-yl) butyl]7-methyl triazolo [1,2,4]–[5,1-b] quinazolin-5(1H)-one and its salts of addition with mineral and organic acids.

5. Process for the preparation of the derivatives as defined by the formula I of claim 1, as well as of their salts, characterized in that: – either a product with the general formula II:

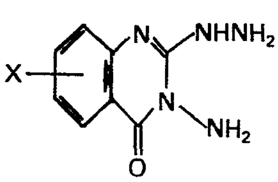

II

in which X is defined as in claim 1, is made to react with an ω-amino alkanol with the general formula III:

$$NH_2-(CH_2)_n-OH \qquad III$$

in which n has the significance indicated in claim 1, so as to obtain a product with the general formula IV:

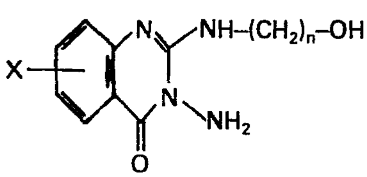

IV

in which n and X have the already indicated significance, which product with the general formula IV is made to react with a formulation agent so as to obtain a product with the general formula V:

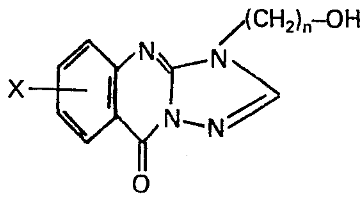

V

in which n and X have the already indicated significance, which product with the general formula V ist submitted to the action of a halogenating agent so as to obtain a product with the general formula VI:

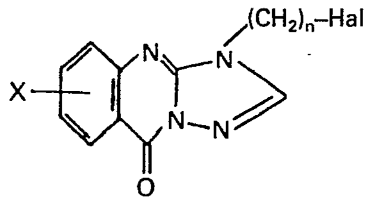

VI

in which n and X have the already indicated significance and Hal represents a chlorine, bromine or iodine atom,

– or a product with the general formula VIII:

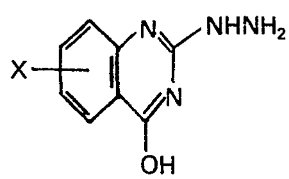

VIII

in which X has the already indicated significance, is made to react with a formylating agent, so as to obtain a product with the general formula IX:

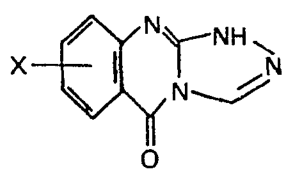

IX

in which X has the already indicated significance, which product with the general formula IX is treated with an alkaline agent so as to obtain a product with the general formula X:

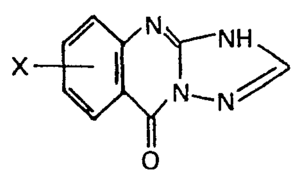

X

in which X has the already indicated significance, which product with the general formula X is made to react with a compound with the general formula XI:

$$Hal_1-(CH_2)_n-Hal_2 \qquad XI$$

in which $Hal_1$ and $Hal_2$, identical or different, each represent a bromine, chlorine or iodine atom and has the already indicated significance, so as to obtain a product with the general formula VI:

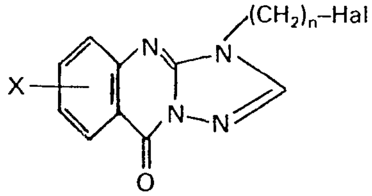

VI

in which n and X have the already indicated significance, and Hal represents a bromine, chlorine or iodine atom, then finally make the product with the general formula VI obtained by starting with the product with the formula II or the formula VIII react with an amine with the general formula VII:

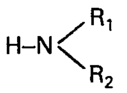

VII

in which $R_1$ and $R_2$ have the significance indicated in claim 1, so as to obtain a product with the general formula I which is isolated, and, if desired, salified.

6. Process according to claim 5, characterized in that:

– the formylating agent which is made to react with the product with the formula IV is dimethyl or diethylacetal of dimethylformamide and the reaction is carried out in toluene, in the presence of paratoluene sulphonic acid:

– the halogenating agent which is made to react with the product with the formula V is a phosphorus halide or thionyl chloride;

– the formylating agent which is made to react with the product with the formula VIII is dimethylformamide, a benzoyl halide, or, when X represents a nitro radical, trimethylorthoformate:

– the alkaline agent utilized to treat the product with the general formula IX is an alkaline hydroxide:

– the reaction of the product with the formula X with the product with the formula XI is carried out in the presence of an alkaline hydride or of an alkaline carbonate, and the reaction is carried out in acetone at reflux:

– the reaction of the product with the formula VI with the amine with the formula VII is carried out at approximately 110 °C, in the presence of an inert solvent, and of a condensation agent.

7. Medicaments, characterized in that they are constituted by the new derivatives of triazoloquinazolinone as defined by the general formula I of claim 1, as well as by their salts of addition with pharmaceutically acceptable acids.

8. Medicaments, characterized in that they are constituted by the new derivatives of triazoloquinazolinone as defined in claim 2 or 3, as well as by their salts of addition with pharmaceutically acceptable acids.

9. Medicaments, characterized in that they are constituted by the new derivatives of triazoloquinazolinone as defined in claim 4.

10. Pharmaceutical compositions, characterized in that they contain, as active principle, one at least of the medicaments as defined in one of the claims 7, 8 or 9.

**Claims for the contracting state: AT**

1. Process for the preparation of the new derivatives of triazoloquinazolinone, as well as of their salts of addition with mineral or organic acids, the said derivatives responding to the general formula I:

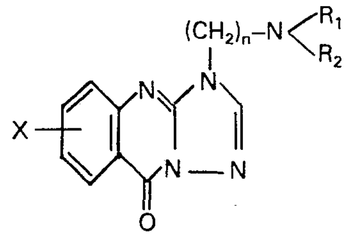

I

in which X represents a hydrogen, fluorine, chlorine or bromine atom, or a nitro, trifluoromethyl, methyl or methoxy radical, n represents a whole number, 2, 3, 4 or 5, $R_1$ and $R_2$ represent a hydrogen atom, an alkyl or hydroxyalkyl radical containing from 1 to 5 carbon atoms, or $R_1$ and $R_2$, together with the nitrogen atom represent a pyrrolidino, piperidino, morpholino or piperazin-1-yl radical, which may be unsubstituted or substituted by one or more hydroxyl, alkyl or hydroxy-alkyl radicals containing from 1 to 5 carbon atoms, cycloalkyl radicals containing from 3 to 6 carbon atoms, formul, acetyl, carbamoyl, thiocarbamoyl, mono or dialkyl carbamoyl or thiocarbamoyl radicals, the alkyl radicals containing from 1 to 6 carbon atoms, alkylsulphonyl radicals, the alkyl radicals containing from 1 to 6 carbon atoms, alkoxycarbonyl radicals containing from 2 to 6 carbon atoms, or phenyl radicals, the said phenyls being themselves un-substituted or substituted by a halogen atom or a trifluoromethyl group, characterized in that:

– either a product with the general formula II:

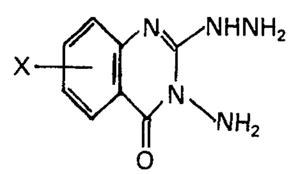

II

in which X is defined as previously, is made to react with an ω-amino alkanol with the general formula III:

$NH_2-(CH_2)_n-OH$ III

in which n has the previously indicated significance, so as to obtain a product with the general formula IV:

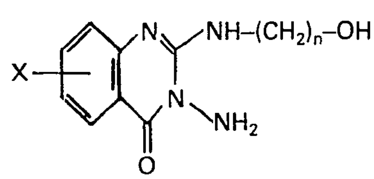

IV

in which n and X have the already indicated significance, which product with the general formula IV is made to react with a formylating agent so as to obtain a product with the general formula V:

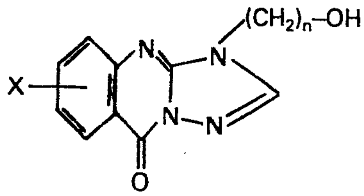

V

in which n and X have the previously indicated significance, which product with the general formula V is submitted to the action of a halogenating agent so as to obtain a product with the general formula VI:

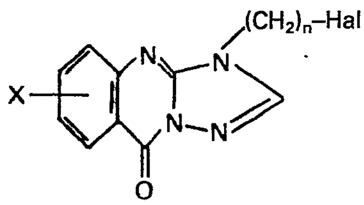

VI

in which n and X have the previously indicated significance and Hal represents a chlorine, bromine or iodine atom,

– or a product with the general formula VIII:

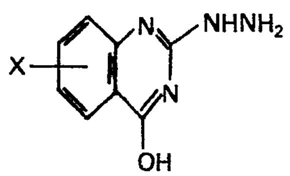

VIII

in which X has the previously indicated significance, is made to react with a formylating agent, so as to obtain a product with the general formula IX:

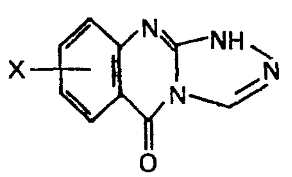

IX

in which X has the previously indicated significance, which product with the general formula IX is treated with an alkaline agent so as to obtain a product with the general formula X:

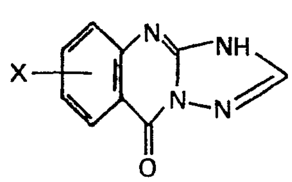

X

in which X has the previously indicated significance, which product with the general formula X is made to react with a compound with the general formula XI:

$Hal_1-(CH_2)_nHal_2$ XI

in which $Hal_1$ and $Hal_2$, identical or different, each represent a bromine, chlorine or iodine atom and n has the previously indicated significance, so as to obtain a product with the general formula VI:

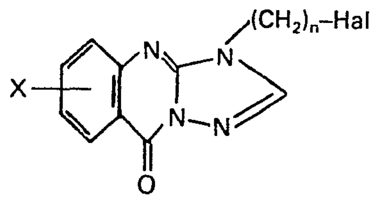

VI

in which n and X have the previously indicated significance, and Hal represents a bromine, chlorine or iodine atom, then finally make the product with the general formula VI obtained by starting with the product with the formula II or the formula VIII react with an amine with the general formula VII:

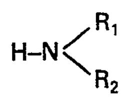

VII

in which $R_1$ and $R_2$ have the previously indicated significance, so as to obtain a product with the general formula I which is isolated, and if desired, salified.

2. Process according to claim 1, characterized in that:

– the formylating agent which is made to react with the product with the formula IV is the dimethyl or the diethylacetal of dimethylformamide and the reaction is carried out in toluene, in the presence of paratoluene sulphonic acid:

– the halogenating agent which is made to react with the product with the formula V is a phosphorus halide or thionyl chloride:

– the formylating agent which is made to react with the product with the formula VIII is dimethylformamide, a halide of benzoyl, or, when X represents a nitro radical, trimethylorthoformate:

– the alkaline agent utilized for treating the product with the general formula IX is an alkaline hydroxide:

– the reaction of the product with the formula X with the product with the formula XI is carried out in the presence of an alkaline hydride or of an alkaline carbonate, and the reaction is carried out in acetone at reflux:

– the reaction of the product with the formula VI with the amine of formula VII is carried out at approximately 110 °C, in the presence of an inert solvent and of a condensation agent.

3. Process according to claim 1 or 2, characterized in that at the start a product is used with the formula II or VIII, in which X represents a hydrogen atom or a methyl or nitro radical, a product with the formula III or XI, in which n represents a whole number, 3, 4 or 5, and a product with the formula VII in which $R_1$ and $R_2$, together with the nitrogen atom to which they are attached, represent a pyrrolidino, piperidino, morpholino or pi-

perazin-1-yl radical, which may be substituted by one or more hydroxyl groups, cycloalkyl groups containing from 3 to 6 carbon atoms, alkoxycarbonyl groups containing from 2 to 6 carbon atoms or phenyl groups, the phenyl radicals being possibly substituted by a halogen atom.

4. Process according to claim 1 or 2, characterized in that at the start a product is used with the formula II or VIII, in which X represents a hydrogen atom or a methyl radical, a product with the formula III or XI, in which n represents a whole number, 3, 4 or 5, and a product with the formula VII in which $R_1$ and $R_2$, together with the nitrogen atom to which they are attached, represent a piperazin-1-yl group of which the second heteroatom is substituted by an ethoxycarbonyl group or by a phenyl group which may be substituted by a chlorine atom.

5. Process according to claim 1 or 2, characterized in that at the start products with the formulae II, III, and VII are used, chosen in such a way that the derivative is prepared corresponding to the formula I of claim 1, of which the name follows:

– 1[4-(4-ethoxycarbonyl piperazin-1-yl) butyl] 7-methyl triazolo [1,2,4] [5,1-b]-quinazolin-5(1H)-one, as well as its salts of addition with mineral or organic acids.